(19) Europäisches Patentamt
European Patent Office
Office européen des brevets

(11) EP 1 687 262 B1

(12) **EUROPEAN PATENT SPECIFICATION**

(45) Date of publication and mention of the grant of the patent:
**19.11.2008 Bulletin 2008/47**

(21) Application number: **04790835.5**

(22) Date of filing: **26.10.2004**

(51) Int Cl.:
***C07C 275/32*** (2006.01) ***C07C 235/38*** (2006.01)
***A61K 31/16*** (2006.01) ***A61K 31/17*** (2006.01)

(86) International application number:
**PCT/EP2004/012050**

(87) International publication number:
**WO 2005/044786 (19.05.2005 Gazette 2005/20)**

(54) **BICYCLIC AMIDE, CARBAMATE OR UREA DERIVATIVES AS VANILLOID RECEPTOR MODULATORS**

BICYCLISCHE AMID-, CARBAMAT- ODER HARNSTOFFDERIVATE ALS MODULATOREN DES VANILLOIDREZEPTORS

DERIVES BICYCLIQUES D'AMIDE, DE CARBAMATE OU D'UREE EN TANT QUE MODULATEURS DU RECEPTEUR VANILLOÏDE

(84) Designated Contracting States:
**DE ES FR GB IT**

(30) Priority: **08.11.2003 EP 03025571**
**22.11.2003 EP 03027003**

(43) Date of publication of application:
**09.08.2006 Bulletin 2006/32**

(73) Proprietor: **Bayer HealthCare AG**
**51368 Leverkusen (DE)**

(72) Inventors:
- **MOGI, Muneto**
  **Tsukuba-shi, Ibaraki-ken 305-0051 (JP)**
- **FUJISHIMA, Hiroshi**
  **Nara-shi, Nara 631-0801 (JP)**
- **TAJIMI, Masaomi**
  **Aichi-ken 478-0044 (JP)**
- **YAMAMOTO, Noriyuki**
  **Osaka-fu 577-0013 (JP)**
- **URBAHNS, Klaus**
  **S-22351 Lund (SE)**
- **HAYASHI, Fumihiko**
  **Tokyo (JP)**
- **TSUKIMI, Yasuhiro**
  **Amagasaki-shi, Hyogo 661-0975 (JP)**
- **GUPTA, Jang**
  **40489 Düsseldorf (DE)**
- **YUASA, Hiroaki**
  **Itano-gunn, Tokushima 771-0205 (JP)**

(56) References cited:
- **TRIVEDI B K ET AL: "A series of conformationally and sterically constrained analogs of N-phenyl-N'-aralkylurea acat inhibitors" BIOORGANIC & MEDICINAL CHEMISTRY LETTERS, OXFORD, GB, vol. 5, no. 19, 5 October 1995 (1995-10-05), pages 2229-2234, XP004135289 ISSN: 0960-894X**
- **HONMA TERUKI ET AL: "Structure-Based Generation of a New Class of Potent Cdk4 Inhibitors: New de Novo Design Strategy and Library Design" JOURNAL OF MEDICINAL CHEMISTRY, AMERICAN CHEMICAL SOCIETY. WASHINGTON, US, vol. 44, 2001, pages 4615-4627, XP002220243 ISSN: 0022-2623**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 687 262 B1

## Description

The present invention relates to a bicyclic amide, carbamate or urea derivative which is useful as an active ingredient of pharmaceutical preparations. The bicyclic amide, carbamate or urea derivative of the present invention has vanilloid receptor (VR1) antagonistic activity, and can be used for the manufacture of a medicament for the prophylaxis and treatment of diseases associated with VR1 activity, in particular for the treatment of urological diseases or disorders, such as detrusor overactivity (overactive bladder), urinary incontinence, neurogenic detrusor oeractivity (detrusor hyperflexia), idiopathic detrusor overactivity (detrusor instability), benign prostatic hyperplasia, and lower urinary tract symptoms; chronic pain, neuropathic pain, postoperative pain, rheumatoid arthritic pain, neuralgia, neuropathies, algesia, nerve injury, ischaemia, neurodegeneration, stroke, and inflammatory disorders such as asthma and chronic obstructive pulmonary (or airways) disease (COPD).

BACKGROUND ART

Vanilloid compounds are characterized by the presence of vanillyl group or a functionally equivalent group. Examples of several vanilloid compounds or vanilloid receptor modulators are vanillin (4-hydroxy-3-methoxy-benzaldehyde), guaiacol (2-methoxy-phenol), zingerone (4-/4-hydroxy-3-methoxyphenyl/-2-butanon), eugenol(2-methoxy4-/2-propenyl/phenol), and capsaicin (8-methy-N-vanillyl-6-noneneamide).

Among others, capsaicin, the main pungent ingredient in "hot" chili peppers, is a specific neurotoxin that desensitizes C-fiber afferent neurons. Capsaicin interacts with vanilloid receptors (VR1), which are predominantly expressed in cell bodies of dorsal root ganglia (DRG) or nerve endings of afferent sensory fibers including C-fiber nerve endings [Tominaga M, Caterina MJ, Malmberg AB, Rosen TA, Gilbert H, Skinner K, Raumann BE, Basbaum AI, Julius D: The cloned capsaicin receptor integrates multiple pain-producing stimuli. Neuron. 21: 531-543, 1998]. The VR1 receptor was recently cloned [Caterina MJ, Schumacher MA, Tominaga M, Rosen TA, Levine JD, Julius D: Nature 389: 816-824, (1997)] and identified as a nonselective cation channel with six transmembrane domains that is structurally related to the TRP (transient receptor potential) channel family. Binding of capsaicin to VR1 allows sodium, calcium and possibly potassium ions to flow down their concentration gradients, causing initial depolarization and release of neurotransmitters from the nerve terminals. VR1 can therefore be viewed as a molecular integrator of chemical and physical stimuli that elicit neuronal signals in pathological conditions or diseases.

There is abundant direct or indirect evidence that shows the relation between VR1 activity and diseases such as pain, ischaemia, and inflammatory disorders (e.g., WO 99/00115 and 00/50387). Further, it has been demonstrated that VR1 transduces reflex signals that are involved in the overactive bladder of patients who have damaged or abnormal spinal reflex pathways [De Groat WC: A neurologic basis for the overactive bladder. Urology 50 (6A Suppl): 36-52, 1997]. Desensitization of the afferent nerves by depleting neurotransmitters using VR1 agonists such as capsaicin has been shown to give promising results in the treatment of bladder dysfunction associated with spinal cord injury and multiple sclerosis [(Maggi CA: Therapeutic potential of capsaicin-like molecules - Studies in animals and humans. Life Sciences 51: 1777-1781, 1992) and (DeRidder D; Chandiramani V; Dasgupta P; VanPoppel H; Baert L; Fowler CJ: Intravesical capsaicin as a treatment for refractory detrusor hyperreflexia: A dual center study with long-term follow-up. J. Urol. 158: 2087-2092, 1997)].

It is anticipated that antagonism of the VR1 receptor would lead to the blockage of neurotransmitter release, resulting in prophylaxis and treatment of the conditions and diseases associated with VR1 activity.

It is therefore expected that antagonists of the VR1 receptor can be used for prophylaxis and treatment of the conditions and diseases including chronic pain, neuropathic pain, postoperative pain, rheumatoid arthritic pain, neuralgia, neuropathies, algesia, nerve injury, ischaemia, neurodegeneration, stroke, inflammatory disorders, urinary incontinence (UI) such as urge urinary incontinence (UUI), and/or overactive bladder.

UI is the involuntary loss of urine. UUI is one of the most common types of UI together with stress urinary incontinence (SUI) which is usually caused by a defect in the urethral closure mechanism. UUI is often associated with neurological disorders or diseases causing neuronal damages such as dementia, Parkinson's disease, multiple sclerosis, stroke and diabetes, although it also occurs in individuals with no such disorders. One of the usual causes of UUI is overactive bladder (OAB) which is a medical condition referring to the symptoms of frequency and urgency derived from abnormal contractions and instability of the detrusor muscle.

There are several medications for urinary incontinence on the market today mainly to help treating UUI. Therapy for OAB is focused on drugs that affect peripheral neural control mechanisms or those that act directly on bladder detrusor smooth muscle contraction, with a major emphasis on development of anticholinergic agents. These agents can inhibit the parasympathetic nerves which control bladder voiding or can exert a direct spasmolytic effect on the detrusor muscle of the bladder. This results in a decrease in intravascular pressure, an increase in capacity and a reduction in the frequency of bladder contraction. Orally active anticholinergic drugs which are commonly prescribed have serious drawbacks such as unacceptable side effects such as dry mouth, abnormal visions, constipation, and

central nervous system disturbances. These side effects lead to poor compliance. Dry mouth symptoms alone are responsible for a 70% non-compliance rate with oxybutynin. The inadequacies of present therapies highlight the need for novel, efficacious, safe, orally available drugs that have fewer side effects.

WO03/014064 discloses the compounds represented by the general formula:

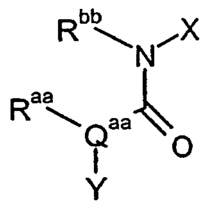

wherein

X represents $C_{3-8}$ cycloalkyl optionally fused by benzene, optionally substituted naphthyl, optionally substituted phenyl, optionally substituted phenyl $C_{1-6}$ straight alkyl, phenyl fused by cycloalykyl, etc;

$Q^{aa}$ represents CH or N;

$R^{aa}$ represents hydrogen or methyl;

$R^{bb}$ represents hydrogen or methyl; and

Y represents substituted naphthyl,

as a vanilloid receptor antagonist.

WO03/022809 discloses the compounds having vanilloid receptor antagonist activity represented by the general formula:

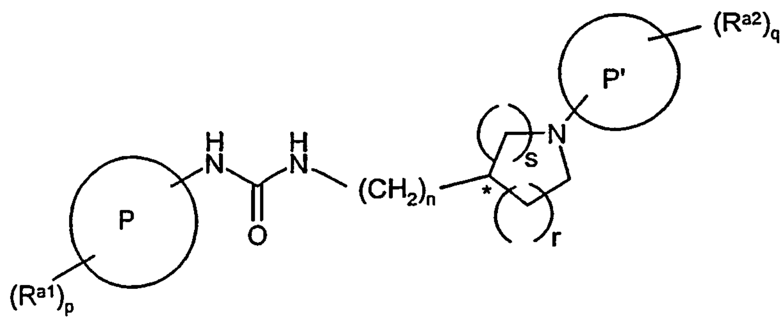

wherein

P and P' independently represent aryl or heteroaryl;
$R^{a1}$ and $R^{a2}$ independently represent hydrogen, alkoxy, hydroxy, etc;

n is 0, 1, 2 or 3; p and q are independently 0,1, 2, 3 or 4; r is 1, 2 or 3; and s is 0, 1 or 2.

WO03/053945 discloses the compounds having vanilloid receptor antagonist activity represented by the general formula:

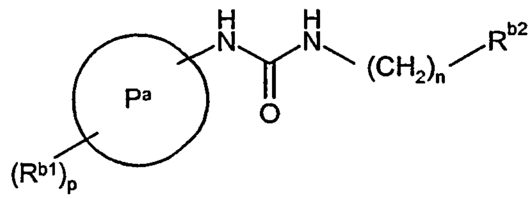

wherein

$P^a$ represents phenyl, naphthyl or heterocyclyl;
n is 2, 3, 4, 5 or 6; p is independently 0,1, 2, 3 or 4;
$R^{b1}$ represents hydrogen, alkoxy, hydroxy, etc; and

$R^{a2}$ represents

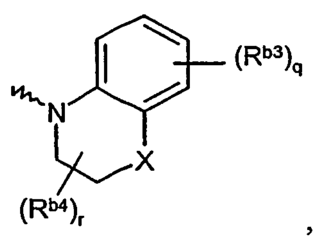

,

wherein X is a bond, C, O, or $NR^{b8}$; and r, q, $R^{b3}$, $R^{b4}$ are defined in the application.

WO03/070247 discloses the compounds having vanilloid receptor antagonist activity represented by the general formula:

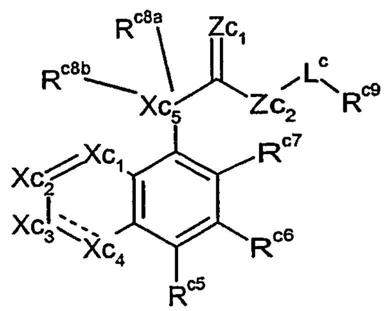

wherein

$Xc_1$ represents N or $CR^{c1}$; $Xc_2$ represents N or $CR^{c2}$; $Xc_3$ represents N, $NR^{c3}$ or $CR^{c3}$; $Xc_4$ represents a bond, N or $CR^{c4}$; $Xc_5$ represents N or C; provided that at least one of $Xc_1$, $Xc_2$, $Xc_3$ and $Xc_4$ is N; $Zc_1$ represents O, NH or S; $Zc_2$ represents a bond, NH or S; $L^c$ represents alkylene, cycloalkylene, etc; $R^{c1}$, $R^{c2}$, $R^{c3}$, $R^{c4}$, $R^{c5}$, $R^{c6}$, $R^{c7}$, $R^{c8a}$ $R^{c8b}$ are defined in the application; and $R^{c9}$ represents hydrogen, aryl, cycloalkyl, and heterocylcle.

WO03/080578 discloses the compounds having vanilloid receptor antagonist activity represented by the general formula:

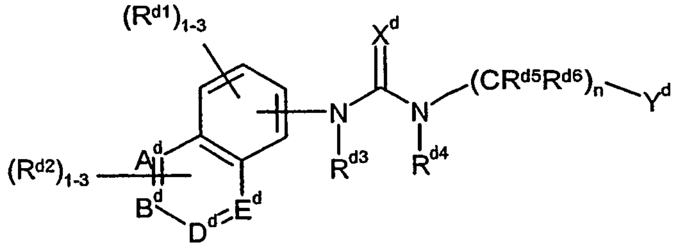

wherein

$A^d$, $B^d$, $D^d$ and $E^d$ are each C or N with the proviso that one or more are N; $X^d$ is an O, S or =NCN; $Y^d$ is an aryl, heteroaryl, carbocyclyl or fused-carbocyclyl; n is 0, 1, 2 or 3; and $R^{d1}$, $R^{d2}$, $R^{d3}$, $R^{d4}$, $R^{d5}$ and $R^{d6}$ are defined in the application.

The development of a compound which has effective VR1 antagonistic activity and can be used for the manufacture of a medicament for the prophylaxis and treatment of diseases associated with VR1 activity, in particular for the treatment of urinary incontinence, urge urinary incontinence, overactive bladder as well as pain, and/or inflammatory diseases such as asthma and COPD has been desired.

SUMMARY OF THE INVENTION

This invention is to provide a bicyclic amide, carbamate or urea derivatives of the formula (I), their tautomeric and stereoisomeric form, and salts thereof:

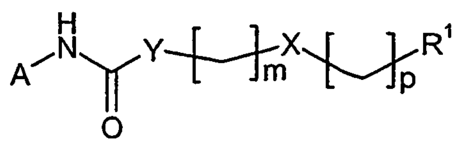

(I)

wherein

A represents

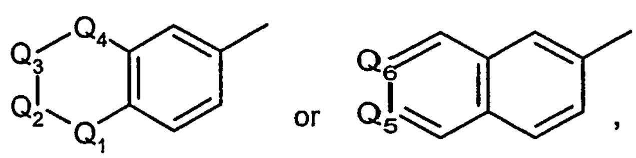

$Q_1$ and $Q_4$ independently represent direct bond or methylene;
$Q_2$ represents $CHR^2$, or CO,
$Q_3$ represents $CHR^3$, or CO,
wherein
$R^2$ represents hydrogen, hydroxy, $C_{1-6}$alkoxy, $C_{1-6}$ alkanoyloxy, or $C_{1-6}$ alkyl optionally substituted by hydroxy, $C_{1-6}$ alkoxy , $C_{1-6}$ alkanoyloxy or mono-, di-, or tri- halogen;
$R^3$ represents hydrogen, hydroxy, $C_{1-6}$ alkoxy, $C_{1-6}$ alkanoyloxy, or $C_{1-6}$ alkyl optionally substituted by hydroxy, $C_{1-6}$ alkoxy, $C_{1-6}$ alkanoyloxy or mono-, di-, or tri- halogen;with the proviso that

$Q_1$ and $Q_4$ can not be direct bond at the same time;

$R^2$ and $R^3$ can not be hydrogen at the same time;

when $Q_1$ represents direct bond,
$R^3$ represents hydroxy, $C_{1-6}$ alkoxy or $C_{1-6}$ alkanoyloxy;

$Q_5$ represents CH or $CR^5$,
wherein
$R^5$ represents hydroxy, $C_{1-6}$ alkoxy, $C_{1-6}$ alkanoyloxy,
or $C_{1-6}$ alkyl optionally substituted by hydroxy, $C_{1-6}$ alkoxy, $C_{1-6}$ alkanoyloxy or mono-, di-, or tri- halogen;
$Q_6$ represents CH or $CR^6$,
wherein
$R^6$ represents hydroxy, $C_{1-6}$ alkoxy, $C_{1-6}$ alkanoyloxy,
or $C_{1-6}$ alkyl optionally substituted by hydroxy, $C_{1-6}$ alkoxy, $C_{1-6}$ alkanoyloxy or mono-, di-, or tri- halogen;
with the proviso that $Q_5$ and $Q_6$ can not be CH at the same time;

m represents an integer from 0 to 3;

p represents an integer 0 or 1;

-X- represents a bond, -O- or $-N(R^4)-$,
wherein
$R^4$ represents hydrogen or $C_{1-6}$ alkyl,
with the proviso that when m is 0, -X- represents a bond; and

-Y- represents $CH_2$, O or NH; and

$R^1$ represents aryl or heteroaryl,
wherein
said aryl and heteroaryl are optionally substituted with one or more substituents independently selected from the group consisting of halogen, nitro, hydroxy, carboxy, cyano, amino, N-($C_{1-6}$alkyl)amino, N,N-di-($C_{1-6}$alkyl)amino, N-($C_{3-8}$ cycloalkyl)amino, $C_{1-6}$alkoxycarbonyl,
sulfonamide, $C_{1-6}$ alkanoyl, N-($C_{1-6}$alkanoyl)amino, carbamoyl, $C_{1-6}$ alkylcarbamoyl, $C_{3-8}$ cycloalkyl, heterocycle, $C_{1-6}$ alkyl [wherein said alkyl is optionally substituted by cyano, nitro, hydroxy, carboxy, amino, $C_{1-6}$ alkoxycarbonyl or mono-, di-, or tri-halogen],
$C_{1-6}$ alkoxy [wherein said alkoxy is optionally substituted by mono-, di-, or tri- halogen],
$C_{1-6}$ alkylthio [wherein said alkylthio is optionally substituted by mono-, di-, or tri- halogen],
phenyl, benzyl and phenoxy ,
[wherein said phenyl, phenyl moiety of said benzyl or phenyl moiety of said phenoxy are optionally substituted by halogen, nitro, hydroxy, carboxy, amino, N-($C_{1-6}$alkyl)amino, N,N-di($C_{1-6}$ alkyl)amino, N-($C_{3-8}$cycloalkyl)amino, $C_{1-6}$ alkoxycarbonyl, $C_{1-6}$alkoxycarbonyl or $C_{1-6}$ alkyl].

In another embodiment, the bicyclic amide, carbamate or urea derivatives of formula (I) can be those wherein;

$Q_1$ and $Q_4$ represent methylene;

$Q_2$ represents $CHR^2$ or CO, wherein
$R^2$ represents hydrogen, hydroxy, $C_{1-6}$ alkoxy, $C_{1-6}$ alkanoyloxy or $C_{1-6}$ alkyl optionally substituted by mono-, di-, or tri- halogen;

$Q_3$ represents $CHR^3$ or CO, wherein
$R^3$ represents hydroxy, $C_{1-6}$ alkoxy, $C_{1-6}$ alkanoyloxy, or $C_{1-6}$ alkyl optionally substituted by mono-, di-, or tri- halogen;

$Q_5$ represents CH;

$Q_6$ represents $CR^6$,
wherein
$R^6$ represents hydroxy, $C_{1-6}$ alkoxy, $C_{1-6}$ alkanoyloxy, or $C_{1-6}$ alkyl optionally substituted by mono-, di-, or tri- halogen;

m represents an integer from 0 to 3;

p represents an integer 0 or 1;

-X- represents a bond, -O- or -N($R^4$)-, wherein $R^4$ represents hydrogen or $C_{1-6}$ alkyl, with the proviso that when m is 0, -X- represents a bond;

-Y- represents $CH_2$, O or NH; and

$R^1$ represents phenyl, naphthyl, pyridyl, or pyrimidyl, wherein said phenyl, naphthyl, pyridyl or pyrimidyl are optionally substituted with one or more substituents independently selected from the group consisting of halogen, nitro, hydroxy, carboxy, cyano, amino, N-($C_{1-6}$alkyl)amino, N,N-di($C_{1-6}$alkyl)amino, N-($C_{3-8}$ cycloalkyl)amino, $C_{1-6}$alkoxycarbonyl, sulfonamide, $C_{1-6}$ alkanoyl, N-($C_{1-6}$alkanoyl)amino, carbamoyl, $C_{1-6}$ alkylcarbamoyl, $C_{3-8}$cycloalkyl, heterocycle, $C_{1-6}$ alkyl [wherein said alkyl is optionally substituted by cyano, nitro, hydroxy, carboxy, amino, $C_{1-6}$ alkoxycarbonyl or mono-, di-, or tri-halogen], $C_{1-6}$ alkoxy [wherein said alkoxy is optionally substituted by mono-, di-, or tri- halogen], $C_{1-6}$ alkylthio [wherein said alkylthio is optionally substituted by mono-, di-, or tri- halogen], phenyl, benzyl and phenoxy, [wherein said phenyl, phenyl moiety of said benzyl or phenyl moiety of said phenoxy are optionally substituted by halogen, nitro, hydroxy, carboxy, amino, N-($C_{1-6}$alkyl)amino, N,N-di($C_{1-6}$ alkyl)amino, N-($C_{3-8}$ cycloalkyl)amino, $C_{1-6}$ alkoxycarbonyl, $C_{1-6}$ alkoxycarbonyl or $C_{1-6}$ alkyl].

In another embodiment, the bicyclic amide, carbamate or urea derivatives of formula (I) can be those wherein;

A represents

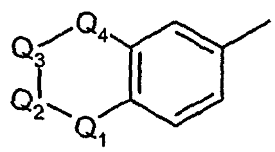

,

$Q_1$ represents methylene;
$Q_4$ represents direct bond;
$Q_2$ represents $CHR^2$ or CO,
wherein
$R^2$ represents hydroxy, $C_{1-6}$ alkoxy or $C_{1-6}$ alkanoyloxy;
$Q_3$ represents $CHR^3$,
wherein
$R^3$ represents hydrogen;
m represents an integer from 0 to 3;
p represents an integer 0 or 1;
-X- represents a bond, -O- or -N($R^4$)-, wherein
$R^4$ represents hydrogen or $C_{1-6}$ alkyl,
with the proviso that when m is 0, -X- represents a bond;
-Y- represents $CH_2$, O or NH; and
$R^1$ represents phenyl, naphthyl, pyridyl, or pyrimidyl,
wherein
said phenyl, naphthyl, pyridyl or pyrimidyl are optionally substituted with one or more substituents independently selected from the group consisting of halogen, nitro, hydroxy, carboxy, cyano, amino, N-($C_{1-6}$alkyl)amino, N,N-di($C_{1-6}$alkyl)amino, N-($C_{3-8}$ cycloalkyl)amino, $C_{1-6}$alkoxycarbonyl, sulfonamide, $C_{1-6}$ alkanoyl, N-($C_{1-6}$alkanoyl)amino, carbamoyl, $C_{1-6}$ alkylcarbamoyl, $C_{3-8}$cycloalkyl, heterocycle,
$C_{1-6}$ alkyl [wherein said alkyl is optionally substituted by cyano, nitro, hydroxy, carboxy, amino, $C_{1-6}$ alkoxycarbonyl or mono-, di-, or tri-halogen],

$C_{1-6}$ alkoxy [wherein said alkoxy is optionally substituted by mono-, di-, or tri- halogen],
$C_{1-6}$ alkylthio [wherein said alkylthio is optionally substituted by mono-, di-, or tri- halogen],
phenyl, benzyl and phenoxy, [wherein said phenyl, phenyl moiety of said benzyl or phenyl moiety of said phenoxy are optionally substituted by halogen, nitro, hydroxy, carboxy, amino, N-($C_{1-6}$alkyl)amino, N,N-di($C_{1-6}$ alkyl)amino, N-($C_{3-8}$ cycloalkyl)amino, $C_{1-6}$ alkoxycarbonyl, $C_{1-6}$ alkoxycarbonyl or $C_{1-6}$ alkyl].

In another embodiment, the bicyclic amide, carbamate or urea derivatives of formula (I) can be those wherein;

A represents

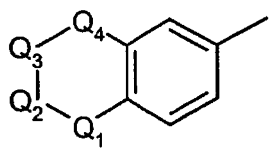

,

$Q_1$ and $Q_4$ represents methylene;
$Q_2$ represents $CHR^2$ ,
wherein
$R^2$ represents hydrogen;
$Q_3$ represents $CHR^3$,
wherein
$R^3$ represents hydrogen, hydroxy, $C_{1-6}$ alkoxy or $C_{1-6}$ alkanoyloxy;

m represents an integer from 0 to 3;

p represents an integer 0 or 1;

-X- represents a bond, -O- or -N($R^4$)-,
wherein $R^4$ is hydrogen or $C_{1-6}$ alkyl,
with the proviso that when m is 0, -X- represents a bond;

-Y- represents $CH_2$, O or NH; and

$R^1$ represents phenyl, naphthyl, pyridyl, or pyrimidyl,
wherein
said phenyl, naphthyl, pyridyl or pyrimidyl are optionally substituted with one or more substituents independently selected from the group consisting of halogen, nitro, hydroxy, carboxy, cyano, amino, N-($C_{1-6}$alkyl)amino, N,N-di($C_{1-6}$alkyl)amino, N-($C_{3-8}$ cycloalkyl)amino, $C_{1-6}$alkoxycarbonyl, sulfonamide, $C_{1-6}$ alkanoyl, N-($C_{1-6}$alkanoyl) amino, carbamoyl, $C_{1-6}$ alkylcarbamoyl, $C_{3-8}$cycloalkyl, heterocycle,
$C_{1-6}$ alkyl [wherein said alkyl is optionally substituted by cyano, nitro, hydroxy, carboxy, amino, $C_{1-6}$ alkoxycarbonyl or mono-, di-, or tri-halogen],
$C_{1-6}$ alkoxy [wherein said alkoxy is optionally substituted by mono-, di-, or tri- halogen],
$C_{1-6}$ alkylthio [wherein said alkylthio is optionally substituted by mono-, di-, or tri- halogen],
phenyl, benzyl and phenoxy ,
[wherein said phenyl, phenyl moiety of said benzyl or phenyl moiety of said phenoxy are optionally substituted by halogen, nitro, hydroxy, carboxy, amino, N-($C_{1-6}$alkyl)-amino, N,N-di($C_{1-6}$ alkyl)amino, N-($C_{3-8}$ cycloalkyl)amino, $C_{1-6}$ alkoxycarbonyl, $C_{1-6}$ alkoxycarbonyl or $C_{1-6}$ alkyl].

In another embodiment, the bicyclic amide, carbamate or urea derivatives of formula (I) can be those wherein;

A represents

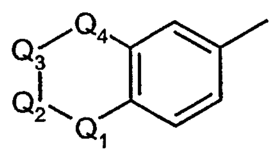

,

$Q_1$ and $Q_4$ represent methylene;

$Q_2$ represents $CHR^2$, wherein $R^2$ represents hydroxy, $C_{1-6}$ alkoxy or $C_{1-6}$ alkanoyloxy;

$Q_3$ represents $CHR^3$, wherein $R^3$ represents hydrogen;

m represents an integer from 1 to 3;

p represents 0 or 1;

-X- represents a bond, -O- or -N($R^4$)-, wherein $R^4$ is hydrogen or $C_{1-6}$ alkyl, with the proviso that when m is 0, -X- represents a bond;

-Y- represents $CH_2$, O or NH; and

$R^1$ represents phenyl, naphthyl, pyridyl, or pyrimidyl, wherein said phenyl, naphthyl, pyridyl or pyrimidyl are optionally substituted with one or more substituents independently selected from the group consisting of halogen, nitro, hydroxy, carboxy, cyano, amino, N-($C_{1-6}$alkyl)amino, N,N-di($C_{1-6}$alkyl)amino, N-($C_{3-8}$ cycloalkyl)ammo, $C_{1-6}$alkoxycarbonyl, sulfonamide, $C_{1-6}$ alkanoyl, N-($C_{1-6}$alkanoyl) amino, carbamoyl, $C_{1-6}$ alkylcarbamoyl, $C_{3-8}$cycloalkyl, heterocycle,
$C_{1-6}$ alkyl [wherein said alkyl is optionally substituted by cyano, nitro, hydroxy, carboxy, amino, $C_{1-6}$ alkoxycarbonyl or mono-, di-, or tri-halogen],
$C_{1-6}$ alkoxy [wherein said alkoxy is optionally substituted by mono-, di-, or tri- halogen],
$C_{1-6}$ alkylthio [wherein said alkylthio is optionally substituted by mono-, di-, or tri- halogen],
phenyl, benzyl and phenoxy,
[wherein said phenyl, phenyl moiety of said benzyl or phenyl moiety of said phenoxy are optionally substituted by halogen, nitro, hydroxy, carboxy, amino, N-($C_{1-6}$alkyl)amino,
N,N-di($C_{1-6}$ alkyl)amino, N-($C_{3-8}$ cycloalkyl)amino, $C_{1-6}$ alkoxycarbonyl, $C_{1-6}$ alkoxycarbonyl or $C_{1-6}$ alkyl].

Preferably, the bicyclic amide, carbamate or urea derivatives of formula (I) can be those wherein;

A represents

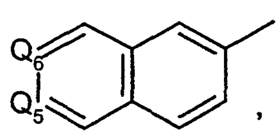

,

$Q_5$ represents CH;
$Q_6$ represent $CR^6$,
wherein
$R^6$ represents hydroxy, $C_{1-6}$alkoxy, $C_{1-6}$ alkanoyloxy, or $C_{1-6}$alkyl optionally substituted by hydroxy, $C_{1-6}$ alkoxy

or $C_{1-6}$ alkanoyloxy;

m represents an integer from 0 to 3;

p represents an integer 0 or 1;

-X- represents a bond, -O- or -N($R^4$)-,
wherein
$R^4$ represents hydrogen or $C_{1-6}$ alkyl,
with the proviso that when m is 0, -X- represents a bond;

-Y- represents NH, O or $CH_2$; and

$R^1$ represents phenyl, naphthyl, pyridyl, or pyrimidyl,
wherein
said phenyl, naphthyl, pyridyl, or pyrimidyl are optionally substituted by one or two of substituents selected from the group consisting of halogen, nitro, $C_{1-6}$alkyl, trifluoro$C_{1-6}$alkyl, $C_{1-6}$alkoxy, trifluoro$C_{1-6}$alkoxy and $C_{1-6}$alkanoylamino.

In another embodiment, the bicyclic amide, carbamate or urea derivatives of formula (I) can be those wherein;

A represents

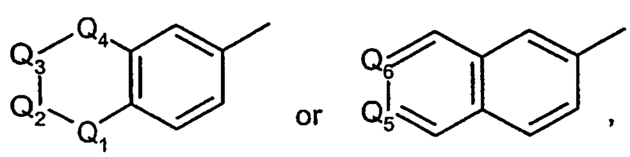

or

,

$Q_1$ and $Q_4$ represents methylene;
$Q_2$ represents $CHR^2$ ,
wherein
$R^2$ represents hydrogen;
$Q_3$ represents $CHR^3$,
wherein
$R^3$ represents hydrogen, hydroxy, $C_{1-6}$ alkoxy or $C_{1-6}$ alkanoyloxy;
$Q_5$ represents CH;
$Q_6$ represents $CR^6$,
wherein
$R^6$ represents hydroxy;
m represents an integer 2;
p represents an integer 0;
-X- represents a bond, -O- or -N($R^4$)-,
wherein
$R^4$ is hydrogen or $C_{1-6}$ alkyl,
with the proviso that when m is 0, -X- represents a bond;
-Y- represents NH; and
$R^1$ represents phenyl, naphthyl, pyridyl, or pyrimidyl,
wherein
said phenyl, naphthyl, pyridyl, or pyrimidyl are optionally substituted by one or two of substituents selected from the group consisting of chloro, bromo, fluoro, nitro, methyl, methoxy, trifluoromethyl, trifluoroethyl, trifluoromethoxy, trifluoro-ethoxy, acetamido and propionylamino;

More preferably, said bicyclic amide, carbamate or urea derivative of the formula (I) is selected from the group consisting of:

N-(7-hydroxy-5,6,7,8-tetrahydronaphthalen-2-yl)-N'-[4-(trifluoromethyl)benzyl]urea;

4-(trifluoromethyl)benzyl(7-hydroxy-5,6,7,8-tetrahydronaphthalen-2-yl)carbamate;

N-(7-hydroxy-5,6,7,8-tetrahydronaphthalen-2-yl)-3-[4-(trifluoromethyl)phenyl]propanamide;

N-(7-hydroxy-5,6,7,8-tetrahydronaphthalen-2-yl)-N'-(2-{[4-(trifluoromethyl)phenyl]-amino}ethyl)urea;

N-(7-hydroxy-5,6,7,8-tetrahydronaphthalen-2-yl)-N'-{2-[4-(trifluoromethyl)phenoxy]-ethyl}urea;

2-{[4-(trifluoromethyl)phenyl]amino}ethyl(7-hydroxy-5,6,7,8-tetrahydronaphthalen-2-yl)-carbamate;

2-[4-(trifluoromethyl)phenoxy]ethyl (7-hydroxy-5,6,7,8-tetrahydronaphthalen-2-yl)carbamate;

N-[4-chloro-3-(trifluoromethyl)phenyl]-N'-(7-hydroxy-5,6,7,8-tetrahydronaphthalen-2-yl)urea;

N-(2-{[4-chloro-3-(trifluoromethyl)phenyl]amino}ethyl)-N'-(7-hydroxy-5,6,7,8-tetra-hydronaphthalen-2-yl)urea;

N-{2-[4-chloro-3-(trifluoromethyl)phenoxy]ethyl}-N'-(7-hydroxy-5,6,7,8-tetrahydro-naphthalen-2-yl)urea;

N-(2-{[4-chloro-3-(trifluoromethyl)phenyl]amino}ethyl)-N'-(6-hydroxy-5,6,7,8-tetra-hydronaphthalen-2-yl)urea; and

N-{2-[4-chloro-3-(trifluoromethyl)phenoxy]ethyl}-N'-(6-hydroxy-5,6,7,8-tetrahydro-naphthalen-2-yl)urea

The bicyclic amide, carbamate or urea derivatives of formula (I), their tautomeric and stereo-isomeric form, and salts thereof surprisingly show excellent VR1 antagonistic activity. They are, therefore suitable especially for the manufacture of a medicament for the prophylaxis and treatment of diseases associated with VR1 activity, in particular for the treatment of urological diseases or disorders, such as detrusor overactivity (overactive bladder), urinary incontinence, neurogenic detrusor oeractivity (detrusor hyperflexia), idiopathic detrusor overactivity (detrusor instability), benign prostatic hyperplasia, and lower urinary tract symptoms.

The compounds of the present invention are also effective for the manufacture of a medicament for treating or preventing a disease selected from the group consisting of chronic pain, neuropathic pain, postoperative pain, rheumatoid arthritic pain, neuralgia, neuropathies, algesia, nerve injury, ischaemia, neurodegeneration and/or stroke, as well as inflammatory diseases such as asthma and COPD since the diseases also relate to VR1 activity.

The compounds of the present invention are also useful for the manufacture of a medicament for the treatment and prophylaxis of neuropathic pain, which is a form of pain often associated with herpes zoster and post-herpetic neuralgia, painful diabetic neuropathy, neuropathic low back pain, posttraumatic and postoperative neuralgia, neuralgia due to nerve compression and other neuralgias, phantom pain, complex regional pain syndromes, infectious or parainfectious neuropathies like those associated with HIV infection, pain associated with central nervous system disorders like multiple sclerosis or Parkinson disease or spinal cord injury or traumatic brain injury, and post-stroke pain.

Furthermore, the compounds of the present invention are useful for the manufacture of a medicament for the treatment of musculoskeletal pain, forms of pain often associated with osteoarthritis or rheumatoid arthritis or other forms of arthritis, and back pain.

In addition, the compounds of the present invention are useful for the manufacture of a medicament for the treatment of pain associated with cancer, including visceral or neuropathic pain associated with cancer or cancer treatment.

The compounds of the present invention are furthermore useful for the manufacture of a medicament for the treatment of visceral pain, e.g. pain associated with obstruction of hollow viscus like gallstone colik, pain associated with irritable bowel syndrome, pelvic pain, vulvodynia, orchialgia or prostatodynia, pain associated with inflammatory lesions of joints, skin, muscles or nerves, and orofascial pain and headache, e.g. migraine or tension-type headache.

Further, the present invention provides a medicament, which includes one of the compounds, described above and optionally pharmaceutically acceptable excipients.

Alkyl per se and "alk" and "alkyl" in alkenyl, alkynyl, alkoxy, alkanoyl, alkylamino, alkylaminocarbonyl, alkylaminosulfonyl, alkylsulfonylamino, alkoxycarbonyl, alkoxycarbonylamino and alkanoylamino represent a linear or branched alkyl radical having generally 1 to 6, preferably 1 to 4 and particularly preferably 1 to 3 carbon atoms, representing illustratively and preferably methyl, ethyl, n-propyl, isopropyl, tert-butyl, n-pentyl and n-hexyl.

Alkoxy illustratively and preferably represents methoxy, ethoxy, n-propoxy, isopropoxy, tert-butoxy, n-pentoxy and n-hexoxy.

Alkylamino illustratively and preferably represents an alkylamino radical having one or two (independently selected) alkyl substituents, illustratively and preferably representing methylamino, ethylamino, n-propylamino, isopropylamino, tert-butylamino, n-pentylamino, n-hexyl-amino, N,N-dimethylamino, N,N-diethylamino, N-ethyl-N-methylamino, N-methyl-N-n-propylamino, N-isopropyl-N-n-propylamino, N-t-butyl-N-methylamino, N-ethyl-N-n-pentylamino and N-n-hexyl-N-methylamino.

Cycloalkyl per se and in cycloalkylamino and in cycloalkylcarbonyl represents a cycloalkyl group having generally 3 to 8 and preferably 5 to 7 carbon atoms, illustratively and preferably representing cyclopropyl, cyclobutyl, cyclopentyl, cyclohexyl and cycloheptyl.

Aryl per se and in arylamino and in arylcarbonyl represents a mono- to tricyclic aromatic carbocyclic radical having generally 6 to 14 carbon atoms, illustratively and preferably representing phenyl, naphthyl and phenanthrenyl.

Heteroaryl per se and the heteroaryl portion of the heteroaralkyl, heteroaryloxy, heteroaralkyloxy, or heteroarylcarbamoyl represent an aromatic mono- or bicyclic radical having generally 5 to 10 and preferably 5 or 6 ring atoms and up to 5 and preferably up to 4 hetero atoms selected from the group consisting of S, O and N, illustratively and preferably representing thienyl, furyl, pyrrolyl, thiazolyl, oxazolyl, imidazolyl, pyridyl, pyrimidyl, pyridazinyl, indolyl, isoindolino, indazolyl, benzofuranyl, benzothiophenyl, quinolinyl, isoquinolinyl, tetrazolyl, and triazolyl.

Heterocyclyl per se and in heterocyclylcarbonyl represents a mono- or polycyclic, preferably mono- or bicyclic, nonaromatic heterocyclic radical having generally 4 to 10 and preferably 5 to 8 ring atoms and up to 3 and preferably up to 2 hetero atoms and/or hetero groups selected from the group consisting of N, O, S, SO and $SO_2$. The heterocyclyl radicals can be saturated or partially unsaturated. Preference is given to 5- to 8-membered monocyclic saturated heterocyclyl radicals having up to two hetero atoms selected from the group consisting of O, N and S, such as illustratively and preferably 1,3-dioxalanyl, tetrahydrofuran-2-yl, pyrrolidin-2-yl, pyrrolidin-3-yl, pyrrolinyl, piperidinyl, morpholinyl, perhydroazepinyl.

EMBODIMENT OF THE INVENTION

The compound of the formula (I) of the present invention can be prepared by combining various known methods. In some embodiments, one or more of the substituents, such as amino group, carboxyl group, and hydroxyl group of the compounds used as starting materials or intermediates are advantageously protected by a protecting group known to those skilled in the art. Examples of the protecting groups are described in "Protective Groups in Organic Synthesis (3rd Edition)" by Greene and Wuts, John Wiley and Sons, New York 1999.

The compound of the formula (I) of the present invention can be, but not limited to be, prepared by Method from [A] to [H] below.

[Method A]

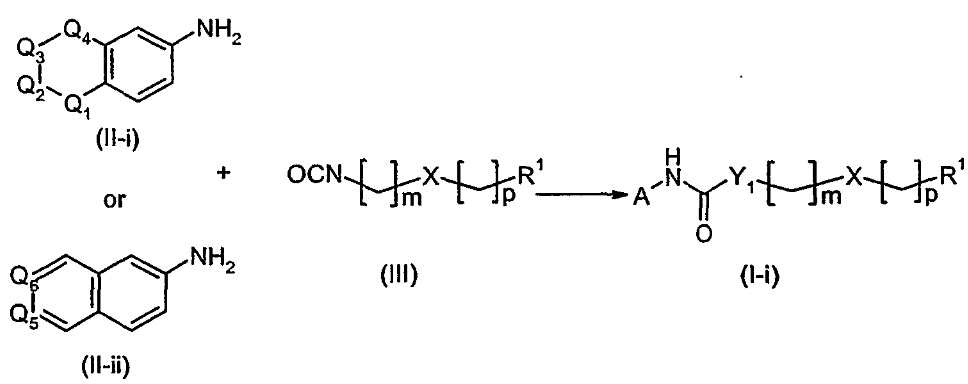

(II-i)

or

(II-ii)

(III)

(I-i)

The compound of the formula (I-i) (wherein m, p, A, $R^1$ and X are the same as defined above and $Y_1$ represents NH) can be prepared by the reaction of the compound of the formula (II-i) (wherein $Q_1$, $Q_2$, $Q_3$ and $Q_4$ are the same as defmed above) or (II-ii) (wherein $Q_5$ and $Q_6$ are the same as defined above) and the compound of the formula (III) (wherein m, p, $R^1$ and X are the same as defined above).

The reaction may be carried out in a solvent including, for instance, halogenated hydrocarbons such as dichloromethane, chloroform and 1,2-dichloroethane; ethers such as diethyl ether, isopropyl ether, dioxane and tetrahydrofuran (THF) and 1,2-dimethoxyethane; aromatic hydrocarbons such as benzene, toluene and xylene; nitriles such as acetonitrile; amides such as N, N-dimethylformamide (DMF), N, N-dimethylacetamide (DMAC) and N-methylpyrrolidone (NMP); urea such as 1,3-dimethyl-2-imidazolidinone (DMI); sulfoxides such as dimethylsulfoxide (DMSO). Optionally, two or more of the solvents selected from the listed above can be mixed and used.

The reaction can be carried out in the presence of organic base such as pyridine or triethylamine.

The reaction temperature can be optionally set depending on the compounds to be reacted. The reaction temperature is usually about room temperature to 100°C. The reaction may be conducted for, usually, 30 minutes to 48 hours and preferably 1 to 24 hours.

The compound (II-i), (II-ii) and (III) can be prepared by the use of known techniques or are commercially available.

[Method B]

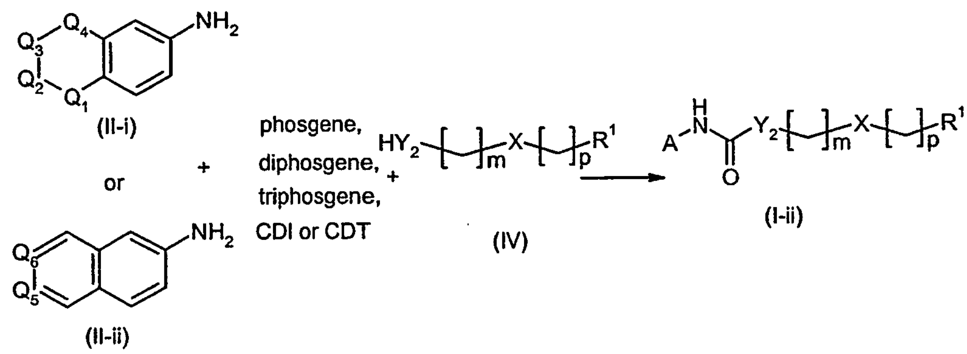


The compound of the formula (I-ii) (wherein m, p, A, $R^1$ and X are the same as defined above and $Y_2$ represents NH or O) can be prepared by reacting the compound of the formula (II-i) (wherein $Q_1$, $Q_2$, $Q_3$ and $Q_4$ are the same as defined above) or (II-ii) (wherein $Q_5$ and $Q_6$ are the same as defined above)with phosgene, diphosgene, triphosgene, 1,1-carbonyldiimidazole (CDI), or 1,1'-carbonyldi(1,2,4-triazole)(CDT), and then adding the compound of the formula (IV) (wherein m, p, $R^1$ and X are the same as defined above and $Y_2$ represents NH or O) to the reaction mixture.

The reaction may be carried out in a solvent including, for instance, halogenated hydrocarbons such as dichloromethane, chloroform and 1,2-dichloroethane; ethers such as diethyl ether, isopropyl ether, dioxane and tetrahydrofuran (THF) and 1,2-dimethoxyethane; aromatic hydrocarbons such as benzene, toluene and xylene; nitriles such as acetonitrile; amides such as N, N-dimethylformamide (DMF), N, N-dimethylacetamide (DMAC) and N-methylpyrrolidone (NMP); urea such as 1,3-dimethyl-2-imidazolidinone (DMI); sulfoxides such as dimethylsulfoxide (DMSO). Optionally, two or more of the solvents selected from the listed above can be mixed and used.

The reaction temperature can be optionally set depending on the compounds to be reacted. The reaction temperature is usually, but not limited to, about 20°C to 50°C. The reaction may be conducted for, usually, 30 minutes to 24 hours and preferably 1 to 10 hours.

The compound (IV) is commercially available or can be prepared by the use of known techniques and phosgene, diphosgene, triphosgene, CDI, and CDT are commercially available.

[Method C]

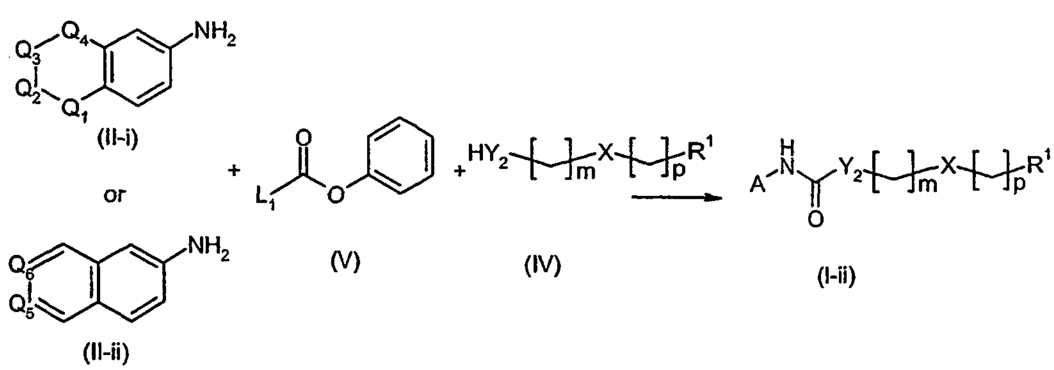

The compound of the formula (I-ii) (wherein m, p, A, $R^1$ and X are the same as defmed above and $Y_2$ represents NH or O) can be also prepared by reacting the compound of the formula (II-i) (wherein $Q_1$, $Q_2$, $Q_3$ and $Q_4$ are the same as defmed above) or (II-ii) (wherein $Q_5$ and $Q_6$ are the same as defined above) with the compound of the formula (V) (wherein $L_1$ represents halogen atom such as chlorine, bromine, or iodine atom) and then adding the compound of the formula (IV) (wherein m, p, $R^1$ and X are the same as defmed above and $Y_2$ represents NH or O) to the reaction mixture.

The reaction may be carried out in a solvent including, for instance, halogenated hydrocarbons such as dichloromethane, chloroform and 1,2-dichloroethane; ethers such as diethyl ether, isopropyl ether, dioxane and tetrahydrofuran (THF) and 1,2-dimethoxyethane; aromatic hydrocarbons such as benzene, toluene and xylene; nitriles such as acetonitrile; amides such as N,N-dimethylformamide (DMF), N, N-dimethylacetamide (DMAC) and N-methylpyrrolidone (NMP); urea such as 1,3-dimethyl-2-imidazolidinone (DMI); sulfoxides such as dimethylsulfoxide (DMSO). Optionally, two or more of the solvents selected from the listed above can be mixed and used.

The reaction temperature can be optionally set depending on the compounds to be reacted. The reaction temperature is usually about 20°C to 50°C. The reaction may be conducted for, usually, 30 minutes to 24 hours and preferably 1 to 10 hours.

The reaction can be advantageously carried out in the presence of a base including, for instance, organic amines such as pyridine, triethylamine and N,N-diisopropylethylamine, dimethylaniline, diethylaniline, 4-dimethylaminopyridine.

The compound (V) is commercially available or can be prepared by the use of known techniques.

[Method D]

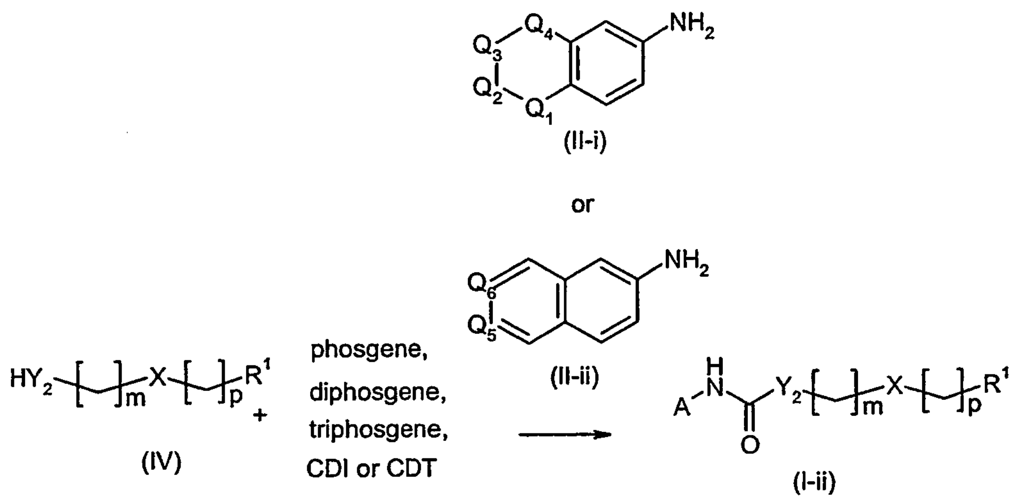


(II-i) or (II-ii) (IV) (I-ii)

The compound of the formula (I-ii) (wherein m, p, A, $R^1$ and X are the same as defined above and $Y_2$ represents NH or O) can be prepared by reacting the compound of the formula (IV) (wherein m, p, $R^1$ and X are the same as defmed above and $Y_2$ represents NH or O) with phosgene, diphosgene, triphosgene, 1,1-carbonyldiimidazole (CDI), or 1,1'-carbonyldi(1,2,4-triazole)(CDT) and then adding the compound of the formula (II-i) (wherein $Q_1$, $Q_2$, $Q_3$ and $Q_4$ are the same as defined above) or (II-ii) (wherein $Q_5$ and $Q_6$ are the same as defined above)to the reaction mixture.

The reaction may be carried out in a solvent including, for instance, halogenated hydrocarbons such as dichloromethane, chloroform and 1,2-dichloroethane; ethers such as diethyl ether, isopropyl ether, dioxane and tetrahydrofuran (THF) and 1,2-dimethoxyethane; aromatic hydrocarbons such as benzene, toluene and xylene; nitriles such as acetonitrile; amides such as N, N-dimethylformamide (DMF), N, N-dimethylacetamide (DMAC) and N-methylpyrrolidone (NMP); urea such as 1,3-dimethyl-2-imidazolidinone (DMI); sulfoxides such as dimethylsulfoxide (DMSO). Optionally, two or more of the solvents selected from the listed above can be mixed and used.

The reaction temperature can be optionally set depending on the compounds to be reacted. The reaction temperature is usually about 20°C to 50°C. The reaction may be conducted for, usually, 30 minutes to 24 hours and preferably 1 to 10 hours.

[Method E]

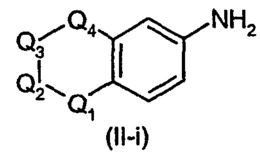

(II-i)

or

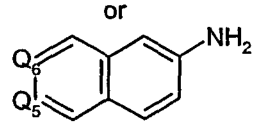

(II-ii)

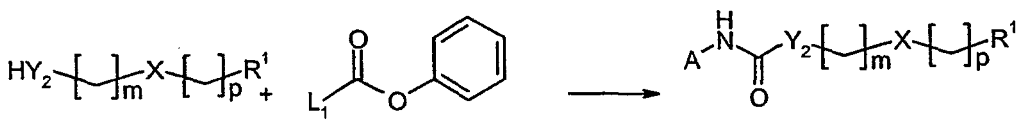

(IV) (V) (I-ii)

The compound of the formula (I-ii) (wherein m, p, A, $R^1$ and X are the same as defined above and $Y_2$ represents NH or O) can be prepared by reacting the compound of the formula (IV) (wherein m, p, $R^1$ and X are the same as defined above and $Y_2$ represents NH or O) with the compound of the formula (V) (wherein $L_1$ is the same as defined above) and then adding the compound of the formula (II-i) (wherein $Q_1$, $Q_2$, $Q_3$ and $Q_4$ are the same as defmed above) or (II-ii) (wherein $Q_5$ and $Q_6$ are the same as defmed above)to the reaction mixture.

The reaction may be carried out in a solvent including, for instance, halogenated hydrocarbons such as dichloromethane, chloroform and 1,2-dichloroethane; ethers such as diethyl ether, isopropyl ether, dioxane and tetrahydrofuran (THF) and 1,2-dimethoxyethane; aromatic hydrocarbons such as benzene, toluene and xylene; nitriles such as acetonitrile; amides such as N, N-dimethylformamide (DMF), N, N-dimethylacetamide (DMAC) and N-methylpyrrolidone (NMP); urea such as 1,3-dimethyl-2-imidazolidinone (DMI); sulfoxides such as dimethylsulfoxide (DMSO). Optionally, two or more of the solvents selected from the listed above can be mixed and used.

The reaction temperature can be optionally set depending on the compounds to be reacted. The reaction temperature is usually about 20°C to 50°C. The reaction may be conducted for, usually, 30 minutes to 24 hours and preferably 1 to 10 hours.

The reaction can be advantageously carried out in the presence of a base including, for instance, organic amines such as pyridine, triethylamine and N,N-diisopropylethylamine, dimethylaniline, diethylaniline, 4-dimethylaminopyridine.

[Method F]

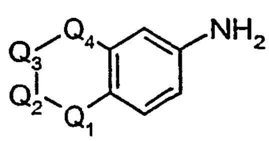

(II-i)

or

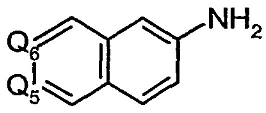

(II-ii)

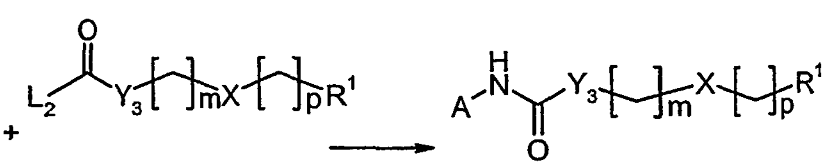

(VI) (I-iii)

The compound of the formula (I-iii) (wherein m, p, A, $R^1$ and X are the same as defined above and $Y_3$ represents $CH_2$) can be prepared by reacting the compound of the formula (II-i) (wherein $Q_1$, $Q_2$, $Q_3$ and $Q_4$ are the same as defined above) or (II-ii) (wherein $Q_5$ and $Q_6$ are the same as defined above) with the compound of the formula (VI) (wherein m, p, $R^1$ and X are the same as defined above, $Y_3$ represents $CH_2$ and wherein $L_2$ represents halogen atom such as chlorine, bromine, or iodine atom, hydroxy or the like).

The reaction may be carried out in a solvent including, for instance, halogenated hydrocarbons such as dichloromethane, chloroform and 1,2-dichloroethane; ethers such as diethyl ether, isopropyl ether, dioxane and tetrahydrofuran (THF) and 1,2-dimethoxyethane; aromatic hydrocarbons such as benzene, toluene and xylene; nitriles such as acetonitrile; amides such as N, N-dimethylformamide (DMF), N, N-dimethylacetamide (DMAC) and N-methylpyrrolidone (NMP); urea such as 1,3-dimethyl-2-imidazolidinone (DMI); sulfoxides such as dimethylsulfoxide (DMSO). Optionally, two or more of the solvents selected from the listed above can be mixed and used.

The reaction temperature can be optionally set depending on the compounds to be reacted. The reaction temperature is usually about 0˚C to 200 ˚C and preferably about 20˚C to 180 ˚C. The reaction may be conducted for, usually, 30 minutes to 48 hours and preferably 2 to 12 hours.

The reaction can be advantageously carried out in the presence of a base including, for instance, organic amines such as pyridine, triethylamine and N,N-diisopropylethylamine, dimethylaniline, diethylaniline, 4-dimethylaminopyridine, and others.

The compound (VI) is commercially available or can be prepared by the use of known techniques.

[Method G]

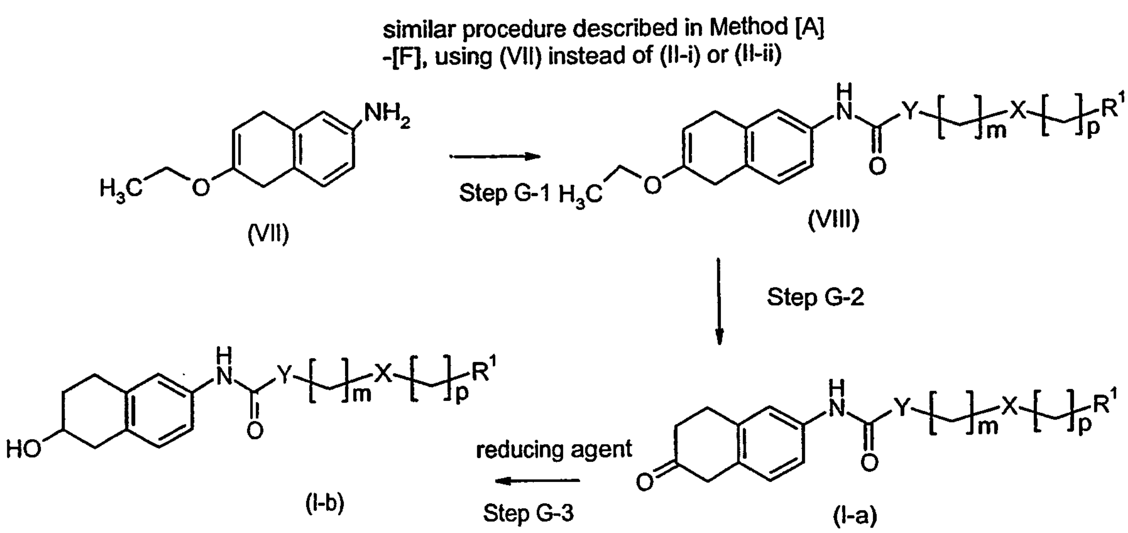


The compound of the formula (I-a) and (I-b) (wherein m, p, $R^1$, X and Y are the same as defined above) can be prepared by the following procedures.

In the Step G-1, the compound of the formula (VIII) (wherein m, p, $R^1$, X and Y are the same as defined above) can be prepared in the similar manner as described in Method from [A] to [F] for the preparation of the compound of the formula (I) by using a compound of the formula (VII) instead of the compound of the formula (II-i) or (II-ii).

In the Step G-2, the compound of the formula (I-a) (wherein m, p, $R^1$, X and Y are the same as defined above) can be prepared by reacting the compound of the formula (VIII) (wherein m, p, $R^1$, X and Y are the same as defined above) with an acid such as hydrochloric acid.

The reaction may be carried out in a solvent including, for instance, halogenated hydrocarbons such as dichloromethane, chloroform and 1,2-dichloroethane; ethers such as diethyl ether, isopropyl ether, dioxane and tetrahydrofuran (THF) and 1,2-dimethoxyethane; alcohols such as methanol, ethanol; water. Optionally, two or more of the solvents selected from the listed above can be mixed and used.

The reaction temperature can be optionally set depending on the compounds to be reacted. The reaction temperature is usually about 20˚C to 100˚C. The reaction may be conducted for, usually, 30 minutes to 24 hours and preferably 1 to 10 hours.

In the Step G-3, the compound of the formula (I-b) (wherein m, p, $R^1$, X and Y are the same as defined above) can be prepared by reacting the compound of the formula (I-a) (wherein m, p, $R^1$, X and Y are the same as defined

above) with reducing agent such as sodium borohydride or lithium aluminum hydride.

The reaction may be carried out in a solvent including, for instance, ethers such as diethyl ether, isopropyl ether, dioxane and tetrahydrofuran (THF) and 1,2-dimethoxyethane; alcohols such as methanol, ethanol, isopropanol. Optionally, two or more of the solvents selected from the listed above can be mixed and used.

The reaction temperature can be optionally set depending on the compounds to be reacted. The reaction temperature is usually about 20°C to 50°C. The reaction may be conducted for, usually, 30 minutes to 24 hours and preferably 1 to 10 hours.

The compound of the formula (VII) is commercially available or can be prepared by the use of known techniques.

[Method H]

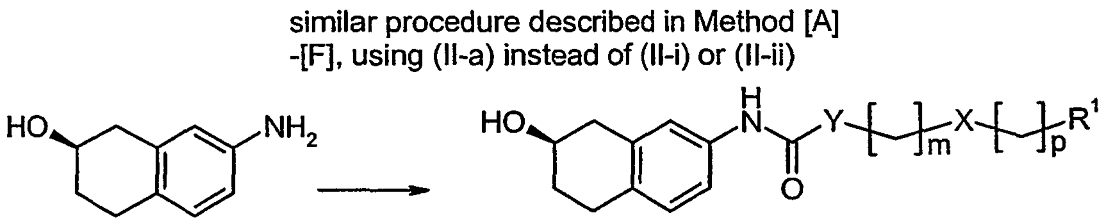


(II-a)

(I-c)

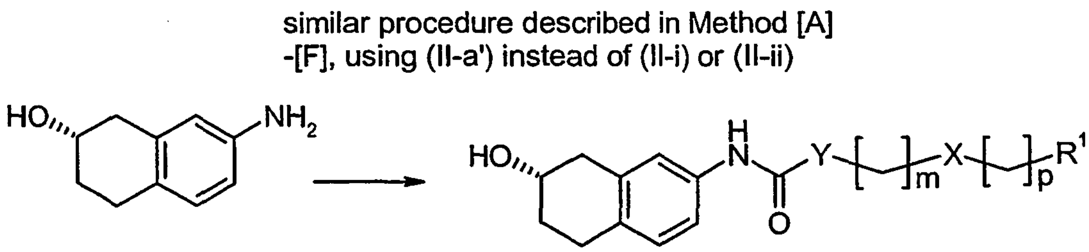


(II-a')

(I-c')

The stereoisomeric form of the compound (I), R form (I-c) (wherein m, p, $R^1$, X and Y are the same as defined above) can be prepared in the similar manner as described in Method from [A] to [F] for the preparation of the compound of the formula (I) by using a compound of the formula (II-a) instead of the compound of the formula (II).

The stereoisomeric form of the compound (I), S form (I-c') (wherein m, p, $R^1$, X and Y are the same as defined above) can be prepared in the similar manner as described in Method from [A] to [F] for the preparation of the compound of the formula (I) by using a compound of the formula (II-a') instead of the compound of the formula (II).

The compound (II-a) or (II-a') can be prepared by the use of known techniques.

When the compound shown by the formula (I) or a salt thereof has an asymmetric carbon in the structure, their optically active compounds and racemic mixtures are also included in the scope of the present invention.

Typical salts of the compound shown by the formula (I) include salts prepared by reaction of the compounds of the present invention with a mineral or organic acid, or an organic or inorganic base. Such salts are known as acid addition and base addition salts, respectively.

Acids to form acid addition salts include inorganic acids such as, without limitation, sulfuric acid, phosphoric acid, hydrochloric acid, hydrobromic acid, hydriodic acid and the like, and organic acids, such as, without limitation, p-toluenesulfonic acid, methanesulfonic acid, oxalic acid, p-bromophenylsulfonic acid, carbonic acid, succinic acid, citric acid, benzoic acid, acetic acid.

Base addition salts include those derived from inorganic bases, such as, without limitation, ammonium hydroxide, alkaline metal hydroxide, alkaline earth metal hydroxides, carbonates, bicarbonates, and the like, and organic bases, such as, without limitation, ethanolamine, triethylamine, tris(hydroxymethyl)aminomethane. Examples of inorganic bases include sodium hydroxide, potassium hydroxide, potassium carbonate, sodium carbonate, sodium bicarbonate, potassium bicarbonate, calcium hydroxide, calcium carbonate.

The compound of the present invention or a salt thereof, depending on its substituents, may be modified to

form lower alkylesters; and/or hydrates or other solvates. Those esters, hydrates, and solvates are included in the scope of the present invention.

The compound of the present invention may be administered in oral forms, such as, without limitation normal and enteric coated tablets, capsules, pills, powders, granules, elixirs, tinctures, solution, suspensions, syrups, solid and liquid aerosols and emulsions. They may also be administered in parenteral forms, such as, without limitation, intravenous, intraperitoneal, subcutaneous, intramuscular, and the like forms, well-known to those of ordinary skill in the pharmaceutical arts. The compounds of the present invention can be administered in intranasal form via topical use of suitable intranasal vehicles, or via transdermal routes, using transdermal delivery systems well-known to those of ordinary skilled in the art.

The dosage regimen with the use of the compounds of the present invention is selected by one of ordinary skill in the arts, in view of a variety of factors, including, without limitation, age, weight, sex, and medical condition of the recipient, the severity of the condition to be treated, the route of administration, the level of metabolic and excretory function of the recipient, the dosage form employed, the particular compound and salt thereof employed.

The compounds of the present invention are preferably formulated prior to administration together with one or more pharmaceutically-acceptable excipients. Excipients are inert substances such as, without limitation carriers, diluents, flavoring agents, sweeteners, lubricants, solubilizers, suspending agents, binders, tablet disintegrating agents and encapsulating material.

Yet another embodiment of the present invention is pharmaceutical formulation comprising a compound of the invention and one or more pharmaceutically-acceptable excipients that are compatible with the other ingredients of the formulation and not deleterious to the recipient thereof. Pharmaceutical formulations of the invention are prepared by combining a therapeutically effective amount of the compounds of the invention together with one or more pharmaceutically-acceptable excipients therefore. In making the compositions of the present invention, the active ingredient may be mixed with a diluent, or enclosed within a carrier, which may be in the form of a capsule, sachet, paper, or other container. The carrier may serve as a diluent, which may be solid, semi-solid, or liquid material which acts as a vehicle, or can be in the form of tablets, pills powders, lozenges, elixirs, suspensions, emulsions, solutions, syrups, aerosols, ointments, containing, for example, up to 10% by weight of the active compound, soft and hard gelatin capsules, suppositories, sterile injectable solutions and sterile packaged powders.

For oral administration, the active ingredient may be combined with an oral, and non-toxic, pharmaceutically-acceptable carrier, such as, without limitation, lactose, starch, sucrose, glucose, sodium carbonate, mannitol, sorbitol, calcium carbonate, calcium phosphate, calcium sulfate, methyl cellulose; together with, optionally, disintegrating agents, such as, without limitation, maize, starch, methyl cellulose, agar bentonite, xanthan gum, alginic acid, and the like; and optionally, binding agents, for example, without limitation, gelatin, natural sugars, beta-lactose, corn sweeteners, natural and synthetic gums, acacia, tragacanth, sodium alginate, carboxymethylcellulose, polyethylene glycol, waxes; and, optionally, lubricating agents, for example, without limitation, magnesium stearate, sodium stearate, stearic acid, sodium oleate, sodium benzoate, sodium acetate, sodium chloride, talc.

In powder forms, the carrier may be a finely divided solid which is in admixture with the finely divided active ingredient. The active ingredient may be mixed with a carrier having binding properties in suitable proportions and compacted in the shape and size desired to produce tablets.

The powders and tablets preferably contain from about 1 to about 99 weight percent of the active ingredient which is the novel composition of the present invention. Suitable solid carriers are magnesium carboxymethyl cellulose, low melting waxes, and cocoa butter.

Sterile liquid formulations include suspensions, emulsions, syrups and elixirs. The active ingredient can be dissolved or suspended in a pharmaceutically acceptable carriers, such as sterile water, sterile organic solvent, or a mixture of both sterile water and sterile organic solvent.

The active ingredient can also be dissolved in a suitable organic solvent, for example, aqueous propylene glycol. Other compositions can be made by dispersing the finely divided active ingredient in aqueous starch or sodium carboxymethyl cellulose solution or in a suitable oil.

The formulation may be in unit dosage form, which is a physically discrete unit containing a unit dose, suitable for administration in human or other mammals. A unit dosage form can be a capsule or tablets, or a number of capsules or tablets. A "unit dose" is a predetermined quantity of the active compound of the present invention, calculated to produce the desired therapeutic effect, in association with one or more excipients. The quantity of active ingredient in a unit dose may be varied or adjusted from 0.1 to 1000 milligrams or more according to the particular treatment involved.

Typical oral dosages of the present invention, when used for the indicated effects, will range from 0.01mg /kg/day to 100 mg/kg/day, preferably from 0.1 mg/kg/day to 30 mg/kg/day, and most preferably from 0.5 mg/kg/day to 10 mg/kg/day. In the case of parenteral administration, it has generally proven advantageous to administer quantities of 0.001 to 100mg /kg/day, preferably from 0.01 mg/kg/day to 1 mg/kg/day. The compounds of the present invention may be administered in a single daily dose, or the total daily dose may be administered in divided doses, two, three, or more times per day. Where delivery is via transdermal forms, of course, administration is continuous.

## EXAMPLES

In the examples below, all quantitative data, if not stated otherwise, relate to percentages by weight.

Mass spectra were obtained using electrospray (ES) ionization techniques (micromass Platform LC). Melting points are uncorrected. Liquid Chromatography - Mass spectroscopy (LC-MS) data were recorded on a Micromass Platform LC with Shimadzu Phenomenex ODS column(4.6 mmΦ X 30 mm) flushing a mixture of acetonitrile-water (9: 1 to 1:9) at 1 ml/min of the flow rate. TLC was performed on a precoated silica gel plate (Merck silica gel 60 F-254). Silica gel (WAKO-gel C-200 (75-150 μm)) was used for all column chromatography separations. All chemicals were reagent grade and were purchased from Sigma-Aldrich, Wako pure chemical industries, Ltd., Great Britain, Tokyo kasei kogyo Co., Ltd., Nacalai tesque, Inc., Watanabe Chemical Ind. Ltd., Maybridge plc, Lancaster Synthesis Ltd., Merck KgaA, Germany, Kanto Chemical Co., Ltd.

$^1H$ NMR spectra were recorded using either Bruker DRX-300 (300 MHz for $^1H$) spectrometer or Brucker 500 UltraShieled™ (500 MHz for 1H). Chemical shifts are reported in parts per million (ppm) with tetramethylsilane (TMS) as an internal standard at zero ppm. Coupling constant (J) are given in hertz and the abbreviations s, d, t, q, m, and br refer to singlet, doblet, triplet, quartet, multiplet, and broad, respectively. The mass determinations were carried out by MAT95 (Finnigan MAT).

All starting materials are commercially available or can be prepared using methods cited in the literature.

The effect of the present compounds was examined by the following assays and pharmacological tests.

[Measurement of capsaicin-induced $Ca^{2+}$ influx in the human VR1-transfected CHO cell line] (Assay 1)

(1) Establishment of the human VR1-CHOluc9aeq cell line

Human vanilloid receptor (hVR1) cDNA was cloned from libraries of axotomized dorsal root ganglia (WO 00/29577). The cloned hVR1 cDNA was constructed with pcDNA3 vector and transfected into a CHOluc9aeq cell line. The cell line contains aequorin and CRE-luciferase reporter genes as read-out signals. The transfectants were cloned by limiting dilution in selection medium (DMEM/F12 medium (Gibco BRL) supplemented with 10% FCS, 1.4 mM Sodium pyruvate, 20 mM HEPES, 0.15% Sodium bicarbonate, 100 U/ml penicillin, 100 μg/ml streptomycin, 2 mM glutamine, non-essential amino acids and 2 mg/ml G418). $Ca^{2+}$ influx was examined in the capsaicin-stimulated clones. A high responder clone was selected and used for further experiments in the project. The human VR1-CHOluc9aeq cells were maintained in the selection medium and passaged every 3-4 days at 1-2.5x$10^5$ cells/flask (75 $mm^2$).

(2) Measurement of $Ca^{2+}$ influx using FDSS-3000

Human VR1-CHOluc9aeq cells were suspended in a culture medium which is the same as the selection medium except for G418 and seeded at a density of 1,000 cells per well into 384-well plates (black walled clear-base / Nalge Nunc International). Following the culture for 48 hrs the medium was changed to 2 μM Fluo-3 AM (Molecular Probes) and 0.02% Puronic F-127 in assay buffer (Hank's balanced salt solution (HBSS), 17 mM HEPES (pH7.4), 1 mM Probenecid, 0.1% BSA) and the cells were incubated for 60 min at 25˚C. After washing twice with assay buffer the cells were incubated with a test compound or vehicle for 20 min at 25˚C. Mobilization of cytoplasmic $Ca^{2+}$ was measured by FDSS-3000 ($\lambda_{ex}$=488nm, $\lambda_{em}$=540nm / Hamamatsu Photonics) for 60 sec after the stimulation with 10 nM capsaicin. Integral R was calculated and compared with controls.

[Measurement of the capsaicin-induced $Ca^{2+}$ influx in primary cultured rat dorsal root ganglia neurons] (Assay 2)

(1) Preparation of rat dorsal root ganglia neurons

New born Wister rats (5-11 days) were sacrificed and dorsal root ganglia (DRG) was removed. DRG was incubated with 0.1% trypsin (Gibco BRL) in PBS(-) (Gibco BRL) for 30 min at 37˚C, then a half volume of fetal calf serum (FCS) was added and the cells were spun down. The DRG neuron cells were resuspended in Ham F12/5% FCS/5% horse serum (Gibco BRL) and dispersed by repeated pipetting and passing through 70 μm mesh (Falcon). The culture plate was incubated for 3 hours at 37˚C to remove contaminating Schwann cells. Non-adherent cells were recovered and further cultured in laminin-coated 384 well plates (Nunc) at 1x$10^4$ cells/50 μl/well for 2 days in the presence of 50 ng/ml recombinant rat NGF (Sigma) and 50 μM 5-fluorodeoxyuridine (Sigma).

(2) $Ca^{2+}$ mobilization assay

DRG neuron cells were washed twice with HBSS supplemented with 17 mM HEPES (pH 7.4) and 0.1% BSA.

After incubating with 2 μM fluo-3AM (Molecular Probe), 0.02% PF127 (Gibco BRL) and 1 mM probenecid (Sigma) for 40 min at 37°C, cells were washed 3 times. The cells were incubated with VR1 antagonists or vehicle (dimethylsulfoxide) and then with 1 μM capsaicin in FDSS-6000 ($\lambda_{ex}$=480nm, $\lambda_{em}$=520nm / Hamamatsu Photonics). The fluorescence changes at 480nm were monitored for 2.5 min. Integral R was calculated and compared with controls.

[Organ bath assay to measure the capsaicin-induced bladder contraction] (Assay 3)

Male Wistar rats (10 week old) were anesthetized with ether and sacrificed by dislocating the necks. The whole urinary bladder was excised and placed in oxygenated Modified Krebs-Henseleit solution (pH 7.4) of the following composition (112mM NaCl, 5.9mM KCl, 1.2mM $MgCl_2$, 1.2mM $NaH_2PO_4$, 2mM $CaCl_2$, 2.5mM $NaHCO_3$, 12mM glucose). Contractile responses of the urinary bladder were studied as described previously [Maggi CA et al: Br.J.Pharmacol. 108: 801-805, 1993]. Isometric tension was recorded under a load of 1 g using longitudinal strips of rat detrusor muscle. Bladder strips were equilibrated for 60 min before each stimulation. Contractile response to 80 mM KCl was determined at 15 min intervals until reproducible responses were obtained. The response to KCl was used as an internal standard to evaluate the maximal response to capsaicin. The effects of the compounds were investigated by incubating the strips with compounds for 30 min prior to the stimulation with 1 μM capsaicin (vehicle: 80% saline, 10% EtOH, and 10% Tween 80). One of the preparations made from the same animal was served as a control while the others were used for evaluating compounds. Ratio of each capsaicin-induced contraction to the internal standard (i.e. KCl-induced contraction) was calculated and the effects of the test compounds on the capsaicin-induced contraction were evaluated.

[Measurement of $Ca^{2+}$ influx in the human P2X1-transfected CHO cell line]

(1) Preparation of the human P2X1-transfected CHOluc9aeq cell line

Human P2X1-transfected CHOluc9aeq cell line was established and maintained in Dulbecco's modified Eagle's medium (DMEM/F12) supplemented with 7.5% FCS, 20 mM HEPES-KOH (pH 7.4), 1.4 mM sodium pyruvate, 100 U/ml penicillin, 100 μg/ml streptomycin, 2 mM glutamine (Gibco BRL) and 0.5 Units/ml apyrase (grade I, Sigma). The suspended cells were seeded in each well of 384-well optical bottom black plates (Nalge Nunc International) at 3 x $10^3$ / 50 μl / well. The cells were cultured for following 48 hrs to adhere to the plates.

(2) Measurement of the intracellular $Ca^{2+}$ levels

P2X1 receptor agonist-mediated increases in cytosolic $Ca^{2+}$ levels were measured using a fluorescent $Ca^{2+}$ chelating dye, Fluo-3 AM (Molecular Probes). The plate-attached cells were washed twice with washing buffer (HBSS, 17 mM HEPES-KOH (pH 7.4), 0.1% BSA and 0.5 units/ml apyrase), and incubated in 40 μl of loading buffer (1 μM Fluo-3 AM, 1 mM probenecid, 1 μM cyclosporin A, 0.01% pluronic (Molecular Probes)in washing buffer) for 1 hour in a dark place. The plates were washed twice with 40 μl washing buffer and 35 μl of washing buffer were added in each well with 5 μl of test compounds or *2 ',3'-o*-(2,4,6-trinitrophenyl) adenosine 5'-triphpsphate (Molecular Probes) as a reference. After further incubation for 10 minutes in dark 200 nM a, β-methylene ATP agonist was added to initiate the $Ca^{2+}$ mobilization. Fluorescence intensity was measured by FDSS-6000 ($\lambda_{ex}$=-410nm, $\lambda_{em}$=510nm / Hamamatsu Photonics) at 250 msec intervals. Integral ratios were calculated from the data and compared with that of a control.

[Measurement of capsaicin-induced bladder contraction in anesthetized rats] (Assay 4)

(1) Animals

Female Sprague-Dawley rats (200~250 g / Charles River Japan) were used.

(2) Catheter implantation

Rats were anesthetized by intraperitoneal administration of urethane (Sigma) at 1.2 g/kg. The abdomen was opened through a midline incision, and a polyethylene catheter (BECTON DICKINSON, PE50) was implanted into the bladder through the dome. In parallel, the inguinal region was incised, and a polyethylene catheter (Hibiki, size 5) filled with 2 IU / ml of heparin (Novo Heparin, Aventis Pharma) in saline (Otsuka) was inserted into a common iliac artery.

(3) Cystometric investigation

The bladder catheter was connected via T-tube to a pressure transducer (Viggo-Spectramed Pte Ltd, DT-XXAD) and a microinjection pump (TERUMO). Saline was infused at room temperature into the bladder at a rate of 2.4 ml/hr. Intravesical pressure was recorded continuously on a chart pen recorder (Yokogawa). At least three reproducible micturition cycles, corresponding to a 20-minute period, were recorded before a test compound administration and used as baseline values.

(4) Administration of test compounds and stimulation of bladder with capsaicin

The saline infusion was stopped before administrating compounds. A testing compound dissolved in the mixture of ethanol, Tween 80 (ICN Biomedicals Inc.) and saline (1 : 1 : 8, v/v/v) was administered intraarterially at 10 mg/kg. 2min after the administration of the compound 10 μg of capsaicin (Nacalai Tesque) dissolved in ethanol was administered intraarterially.

(5) Analysis of cystometry parameters

Relative increases in the capsaicin-induced intravesical pressure were analyzed from the cystometry data. The capsaicin-induced bladder pressures were compared with the maximum bladder pressure during micturition without the capsaicin stimulation. The testing compounds-mediated inhibition of the increased bladder pressures was evaluated using Student's t-test. A probability level less than 5% was accepted as significant difference.

[Measurement of over active bladder in anesthetized cystitis rats] (Assay 5)

(1) Animals

Female Sprague-Dawley rats (180~250 g / Charles River Japan) were used. Cyclophosphamide (CYP) dissolved in saline was administered intraperitoneally at 150 mg/kg 48 hours before experiment.

(2) Catheter implantation

Rats were anesthetized by intraperitoneal administration of urethane (Sigma) at 1.25 g/kg. The abdomen was opened through a midline incision, and a polyethylene catheter (BECTON DICKINSON, PE50) was implanted into the bladder through the dome. In parallel, the inguinal region was incised, and a polyethylene catheter (BECTON DICKINSON, PE50) filled with saline (Otsuka) was inserted into a femoral vein. After the bladder was emptied, the rats were left for 1 hour for recovery from the operation.

(3) Cystometric investigation

The bladder catheter was connected via T-tube to a pressure transducer (Viggo-Spectramed Pte Ltd, DT-XXAD) and a microinjection pump (TERUMO). Saline was infused at room temperature into the bladder at a rate of 3.6 ml/hr for 20 min. Intravesical pressure was recorded continuously on a chart pen recorder (Yokogawa). At least three reproducible micturition cycles, corresponding to a 20-minute period, were recorded before a test compound administration.

(4) Administration of test compounds

A testing compound dissolved in the mixture of ethanol, Tween 80 (ICN Biomedicals Inc.) and saline (1 : 1 : 8, v/v/v) was administered intravenously at 0.05 mg/kg, 0.5 mg/kg or 5 mg/kg. 3min after the administration of the compound, saline (Nacalai Tesque) was infused at room temperature into the bladder at a rate of 3.6 ml/hr.

(5) Analysis of cystometry parameters

The cystometry parameters were analyzed as described previously [ Lecci A et al: Eur. J. Pharmacol. 259: 129-135, 1994]. The micturition frequency calculated from micturition interval and the bladder capacity calculated from a volume of infused saline until the first micturition were analyzed from the cystometry data. The testing compounds-mediated inhibition of the frequency and the testing compounds-mediated increase of bladder capacity were evaluated using unpaired Student's t-test. A probability levels less than 5% was accepted as significant difference. Data were analyzed as the mean ± SEM from 4 - 7 rats.

[Measurement of Acute Pain]

Acute pain is measured on a hot plate mainly in rats. Two variants of hot plate testing are used: In the classical variant animals are put on a hot surface (52 to 56 ˚C) and the latency time is measured until the animals show nociceptive behavior, such as stepping or foot licking. The other variant is an increasing temperature hot plate where the experimental animals are put on a surface of neutral temperature. Subsequently this surface is slowly but constantly heated until the animals begin to lick a hind paw. The temperature which is reached when hind paw licking begins is a measure for pain threshold. Compounds are tested against a vehicle treated control group. Substance application is performed at different time points via different application routes (i.v., i.p., p.o., i.t., i.c.v., s.c., intradermal, transdermal) prior to pain testing.

[Measurement of Persistent Pain]

Persistent pain is measured with the formalin or capsaicin test, mainly in rats. A solution of 1 to 5% formalin or 10 to 100 μg capsaicin is injected into one hind paw of the experimental animal. After formalin or capsaicin application the animals show nociceptive reactions like flinching, licking and biting of the affected paw. The number of nociceptive reactions within a time frame of up to 90 minutes is a measure for intensity of pain.

Compounds are tested against a vehicle treated control group. Substance application is performed at different time points via different application routes (i.v., i.p., p.o., i.t., i.c.v., s.c., intradermal, transdermal) prior to formalin or capsaicin administration.

[Measurement of Neuropathic Pain]

Neuropathic pain is induced by different variants of unilateral sciatic nerve injury mainly in rats. The operation is performed under anesthesia. The first variant of sciatic nerve injury is produced by placing loosely constrictive ligatures around the common sciatic nerve (Bennett and Xie, Pain 33 (1988): 87-107). The second variant is the tight ligation of about the half of the diameter of the common sciatic nerve (Seltzer et al., Pain 43 (1990): 205-218). In the next variant, a group of models is used in which tight ligations or transections are made of either the L5 and L6 spinal nerves, or the L5 spinal nerve only (KIM SH; CHUNG JM, AN EXPERIMENTAL-MODEL FOR PERIPHERAL NEUROPATHY PRODUCED BY SEGMENTAL SPINAL NERVE LIGATION IN THE RA, PAIN 50 (3) (1992): 355-363). The fourth variant involves an axotomy of two of the three terminal branches of the sciatic nerve (tibial and common peroneal nerves) leaving the remaining sural nerve intact whereas the last variant comprises the axotomy of only the tibial branch leaving the sural and common nerves uninjured. Control animals are treated with a sham operation.

Postoperatively, the nerve injured animals develop a chronic mechanical allodynia, cold allodynia, as well as a thermal hyperalgesia. Mechanical allodynia is measured by means of a pressure transducer (electronic von Frey Anesthesiometer, IITC Inc.-Life Science Instruments, Woodland Hills, SA, USA; Electronic von Frey System, Somedic Sales AB, Hörby, Sweden). Thermal hyperalgesia is measured by means of a radiant heat source (Plantar Test, Ugo Basile, Comerio, Italy), or by means of a cold plate of 5 to 10 ˚C where the nocifensive reactions of the affected hind paw are counted as a measure of pain intensity. A further test for cold induced pain is the counting of nocifensive reactions, or duration of nocifensive responses after plantar administration of acetone to the affected hind limb. Chronic pain in general is assessed by registering the circadanian rhytms in activity (Surjo and Arndt, Universität zu Köln, Cologne, Germany), and by scoring differences in gait (foot print patterns; FOOTPRINTS program, Klapdor et al., 1997. A low cost method to analyse footprint patterns. J. Neurosci. Methods 75, 49-54).

Compounds are tested against sham operated and vehicle treated control groups. Substance application is performed at different time points via different application routes (i.v., i.p., p.o., i.t., i.c.v., s.c., intradermal, transdermal) prior to pain testing.

[Measurement of Inflammatory Pain]

Inflammatory pain is induced mainly in rats by injection of 0.75 mg carrageenan or complete Freund's adjuvant into one hind paw. The animals develop an edema with mechanical allodynia as well as thermal hyperalgesia. Mechanical allodynia is measured by means of a pressure transducer (electronic von Frey Anesthesiometer, IITC Inc.-Life Science Instruments, Woodland Hills, SA, USA). Thermal hyperalgesia is measured by means of a radiant heat source (Plantar Test, Ugo Basile, Comerio, Italy, Paw thermal stimulator, G. Ozaki, University of California, USA). For edema measurement two methods are being used. In the first method, the animals are sacrificed and the affected hindpaws sectioned and weighed. The second method comprises differences in paw volume by measuring water displacement in a plethysmometer (Ugo Basile, Comerio, Italy).

Compounds are tested against uninflamed as well as vehicle treated control groups. Substance application is performed at different time points via different application routes (i.v., i.p., p.o., i.t., i.c.v., s.c., intradermal, transdermal)

prior to pain testing.

[Measurement of Diabetic Neuropathic Pain]

Rats treated with a single intraperitoneal injection of 50 to 80 mg/kg streptozotocin develop a profound hyperglycemia and mechanical allodynia within 1 to 3 weeks. Mechanical allodynia is measured by means of a pressure transducer (electronic von Frey Anesthesiometer, IITC Inc.-Life Science Instruments, Woodland Hills, SA, USA).

Compounds are tested against diabetic and non-diabetic vehicle treated control groups. Substance application is performed at different time points via different application routes (i.v., i.p., p.o., i.t., i.c.v., s.c., intradermal, transdermal) prior to pain testing.

Results in capsaicin-induced $Ca^{2+}$ influx assay in the human VR1-transfected CHO cell line (Assay 1) are shown in the Examples below. For practical reasons, the compounds are grouped in four classes based on activity as follows:

$$IC_{50} = A\ (< \text{or} =)\ 0.1\mu M < B\ (< \text{or} =)\ 0.5\ \mu M < C\ (< \text{or} =)\ 1\ \mu M < D$$

The compounds of the present invention also show excellent selectivity, and strong activity in other assays 2-5 and assays for pain described above.

**Preparation of starting compounds**

**[Starting compound A]**

**7-amino-1,2,3,4-tetrahydronaphthalen-2-ol**

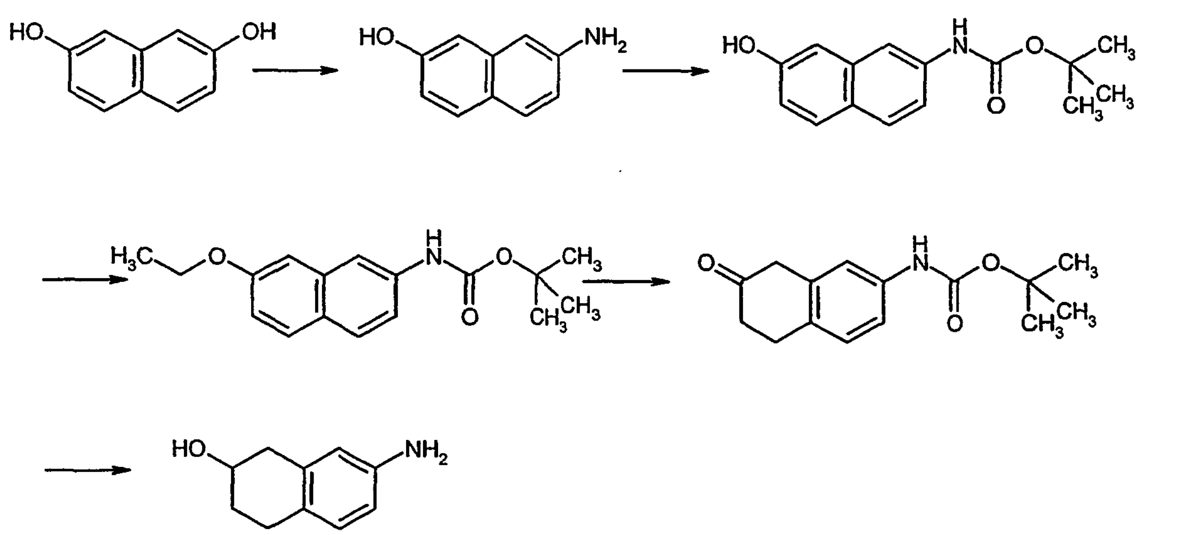

A mixture of 2,7-dihydroxynaphthalene (3.24 g, 20.2 mmol), sodium bisulfite (2.38 g, 22.9 mmol), and 28 % aq. ammonia solution (50 mL) in sealed stainless-steel reactor was heated at 150 ˚C for 4.5 hours. After cooled to ambient temperature, a mixture of ethylacetate and acetonitrile was added and then extracted aqueous 1N sodium hydroxide solution. The aqueous layer was acidified to pH 1, and the mixture was extracted with ethylacetate. The mixture was concentrated under reduced pressure, and the obtained residue was purified by silica gel column chromatography (eluent: ethylacetate / hexane = 1 / 3) to provide 7-amino-2-naphthol (0.54 g).
MS (ESI) m/z 160 $[M+H]^+$
$^1H$ NMR (CDCl3-*d*) δ 3.80 (brs, 2H), 4.82 (s, 1H), 6.78 (d, *J* = 8.5 Hz, 1H), 7.26 - 6.83 (m, 2H), 6.90 (s, 1H), 7.57 (d, *J* = 7.8 Hz, 1H), 7.58 (d, *J* = 7.8 Hz, 1H).

Next, a mixture of 7-amino-2-naphthol (0.54 g, 3.39 mmol) and di-*t*-butylcarbonate (0.74g, 3.39 mmol) in tetrahydrofuran (30 mL) was stirred at room temperature for 16 hours. To the mixture was added water and extracted with ethylacetate. The organic layer was dried over $MgSO_4$, filtered, and concentrated under reduced pressure. The obtained residue was purified by column chromatography (eluent: ethylacetate / hexane = 1 / 2) to afford *tert*-butyl (7-hydroxy-2-naphthyl)carbamate (615 mg).
MS (ESI) m/z 204 $[M+H]^+$
$^1H$ NMR (CDCl3-*d*) δ 1.53 (s, 9H), 5.22 (s, 1H), 6.59 (brs, 1H), 6.98 (dd, J= 2.5, 8.8 Hz, 1H), 7.05 (d, *J* = 2.5 Hz, 1H), 7.14 (dd, *J* = 2.1, 8.8 Hz, 1H), 7.64 (d, *J* = 8.7 Hz, 1H), 7.66 (d, *J* = 8.7 Hz, 1H), 7.84 (s, 1H).

Next, a mixture of *tert*-butyl (7-hydroxy-2-naphthyl)carbamate (610 mg, 2.35 mmol), ethyliodide (734 mg, 4.70 mmol), and potassium carbonate (650 mg, 4.70 mmol) in acetone (50 mL) was stirred at refluxing temperature for 16 hours. The mixture was filtered and the filtrate was concentrated under reduced pressure. The obtained residue was purified by silica gel column chromatography (eluent: ethylacetate/hexane = 1 / 2) to give *tert*-butyl (7-ethoxy-2-naphthyl)-carbamate (440 mg).

MS (ESI) m/z 232 $[M+H]^+$
$^1H$ NMR (CDCl3-*d*) δ1.46 (t, *J* = 7.0 Hz, 3H), 1.55 (s, 9H), 4.12 (q, *J* = 7.0 Hz, 2H), 6.58 (s, 1H), 7.02 (dd, *J* = 2.5, 8.9 Hz, 1H), 7.06 (d, *J* = 2.3 Hz, 1H), 7.12 (dd, *J* = 2.3, 8.9 Hz, 1H), 7.63 (d, *J*= 8.9 Hz, 1H), 7.65 (d, *J* = 8.9 Hz, 1H), 7.92 (s, 1H).

Next, in a flask containing *tert*-butyl (7-ethoxy-2-naphthyl)carbamate (530 mg, 1.84 mmol) and *t*-buthanol (0.53 mL) in tetrahydrofuran (25 mL) was collected liquid ammonia (150 mL) at -78 ˚C. Lithium (38.4 mg) was added, and the mixture was stirred for 1 hour. After raising to room temperature, the mixture was concentrated under reduced pressure then water was added. The mixture was extracted with ethylacetate, and the organic layer was washed with brine, dried over $MgSO_4$, filtered and concentrated under reduced pressure. To the obtained residue was added a mixture of tetrahydrofuran (38 mL) and aqueous 1N solution of hydrochloric acid (12 mL) and stirred at 45 ˚C for 45 minutes. The mixture was concentrated under reduced temperature and then ethylacetate was added. The organic layer was dried over $MgSO_4$, filtered, and concentrated under reduced pressure. The resulting material was purified by silica gel column chromatography (eluent: ethylacetate / hexane = 1 / 4) to provide *tert*-butyl (7-oxo-5,6,7,8-tetrahydronaphthalen-2-yl) carbamate (177 mg).
MS (ESI) m/z 206 $[M+H]^+$
$^1H$ NMR (CDCl3-*d*) δ 1.52 (s, 9H), 2.53 (t, *J* = 6.9 Hz, 2H), 3.00 (t, *J* = 6.9 Hz, 2H), 3.56 (s, 2H), 4.30 (brs, 1H), 6.41 (brs, 1H), 7.10 - 7.15 (m, 2H).

Next, to a solution of *tert*-butyl (7-oxo-5,6,7,8-tetrahydronaphthalen-2-yl)carbamate (177 mg, 0.68 mmol) in methanol (10 mL) was added sodium borohydride (25.6 mg, 0.68 mml) at 0 ˚C. After stirred for 1 hour, water was added and the mixture was concentrated under reduced pressure. To the resulting residue was added 4N hydrochloric acid in 1,4-dioxane solution, and the mixture was stirred for 3.5 hours. After neutralized with saturated aqueous solution of sodium bicarbonate, the mixture was extracted with chloroform. The organic layer was dried over $MgSO_4$, filtered, and concentrated under reduced pressure. The obtained residue was recrystallized from diethylether to afford 7-amino-1,2,3,4-tetrahydronaphthalen-2-ol (78.1 mg).

MS (ESI) m/z 164 $[M+H]^+$
$^1H$ NMR (acetone-*d6*) δ1.69 - 1.75 (m, 2H), 2.64 - 2.95 (m, 5H), 3.60 - 3.80 (m, 1H), 4.02 (brs, 1H), 6.39 (d, *J* = 6.0 Hz, 1H), 6.41 (dd, *J* = 6.0, 8.0 Hz, 1H), 6.96 (d, *J* = 8.0 Hz, 1H).

**[Starting compound B]**

**7-Amino-1,2,3,4-tetrahydro-naphthalen-2-ol (enantiomer)**

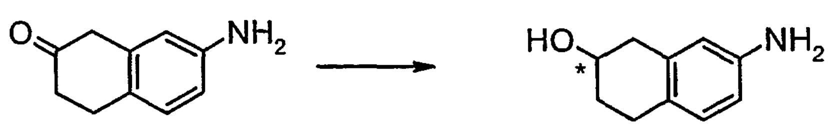

To a stirred solution of benzeneruthenium(II) chloride dimer and (1S, 2R)-(-)-cis-1-amino-2-indanol in degaussed isopropanol was heated at 80˚C for 20 minutes under argon. The mixture was added to the solution of 7-amino-3,4-dihydro-1H-naphthalen-2-one in isopropanol at room temperature. A solution of potassium hydroxide in isopropanol was added, and the mixture was stirred at 45˚C for 1 hour. The mixture was passed through silica gel and washed with ethylacetate. The filtrate was concentrated under reduced pressure to afford 7-amino-1,2,3,4-tetra-hydro-naphthalen-2-ol enantiomer.

The other enantiomer of 7-amino-1,2,3,4-tetrahydronaphthalen-2-ol was obtained in the same fashion replacing (1S,2R)-(-)-cis-1-amino-2-indanol with (1R,2S)-(+)-cis-1-amino-2-indanol.

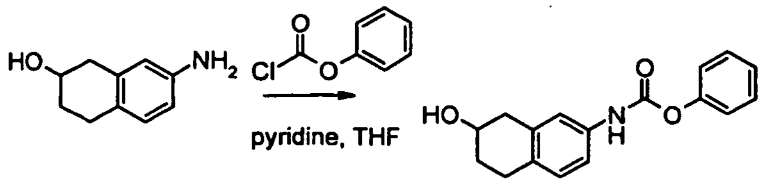


To a stirred solution of 7-amino-1,2,3,4-tetrahydro-naphthalen-2-ol and pyridine in THF was added phenyl chloroformate, and the mixture was stirred for 1 hour at room temperature. To the product mixture was added water and extracted with ethylacetate. The organic layer was washed with brine, dried over $Na_2SO_4$, filtered and concentrated under reduced pressure. The obtained residue was triturated with ethylacetate and hexane to afford (7-hydroxy-5,6,7,8-tetrahydro-naphthalen-2-yl)-carbamic acid phenyl ester.

**Example 1-1**

N-[4-chloro-3-(trifluoromethyl)phenyl]-N'-(7-hydroxy-5,6,7,8-tetrahydronaphthalen-2-yl)urea

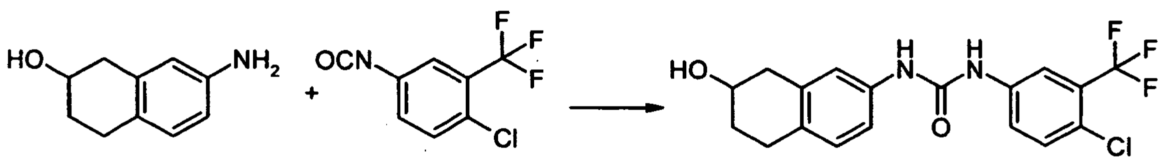

A mixture of 7-amino-1,2,3,4-tetrahydronaphthalen-2-ol (70.0 mg, 0.43 mmol) and 4-chloro-3-trifluoromethylphenyl isocyanate (95.0 mg, 0.43 mmol) in *N,N*-dimethylformamide (10 mL) was stirred at 50°C for 2 hours. After the mixture was concentrated under reduced pressure, the obtained residue was purified by silica gel column chromatography (eluent: ethylacetate / hexane = 2.5/1) to provide N-[4-chloro-3-(trifluoromethyl)phenyl]-N'-(7-hydroxy-5,6,7,8-tetrahydronaphthalen-2-yl)urea (49.9 mg).
MS (ESI) m/z 385 $[M+H]^+$
$^1H$ NMR (DMSO-*d6*) δ 1.59 - 1.63 (m, 1H), 1.80 - 1.88 (m, 1H), 2.56 (dd, *J* = 7.9, 16.1 Hz, 1H), 2.65 (dq, *J* = 9.5, 16.7 Hz, 1H), 2.78 (dt, *J* = 5.4, 16.7 Hz, 1H), 2.89 (dd, *J* = 5.4, 16.1 Hz, 1H), 3.89 (m, 1H), 4.73 (d, *J* = 6.0 Hz, 1H), 5.97 (d, *J* = 8.2 Hz, 1H), 7.13 (dd, *J* = 2.3, 8.2 Hz, 1H), 7.16 (d, *J* = 2.3, 1H), 7.58 - 7.62 (m, 2H), 8.10 (d, *J* = 2.0 Hz, 1H), 8.62 (s, 1H), 9.06 (s, 1H).

Also, the following compounds (as examples and comparative examples)are prepared in a similar manner.

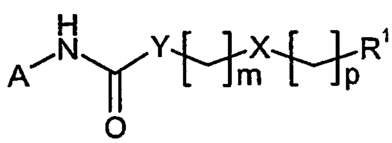

| Example | A | m | p | -X- | Y | -R1 |
|---|---|---|---|---|---|---|
| **1-2** | 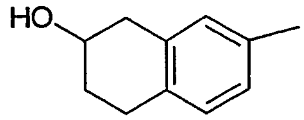 | 0 | 0 | bond | N | 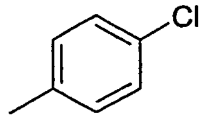 |
| **1-3** | 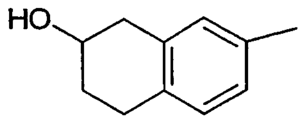 | 1 | 0 | bond | C | 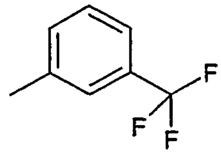 |
| **1-4** | 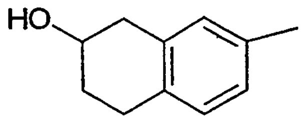 | 0 | 1 | bond | C | 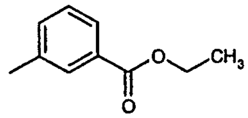 |
| **1-5** | 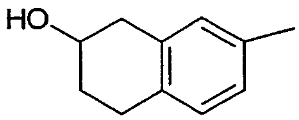 | 0 | 1 | -O- | C | 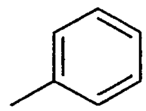 |
| **1-6** | 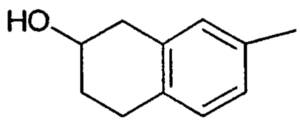 | 0 | 0 | -O- | C | 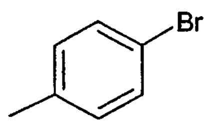 |
| **1-7** comparative | 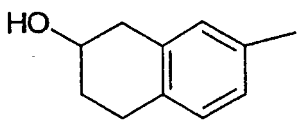 | 0 | 2 | -NH- | C | 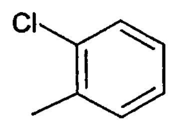 |
| **1-8** comparative | 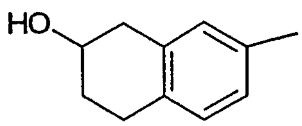 | 0 | 2 | -NH- | N | 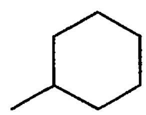 |
| **1-9** | 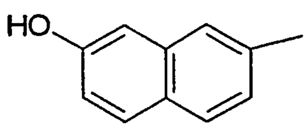 | 0 | 0 | bond | C | 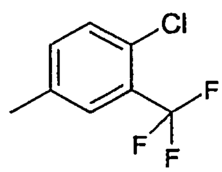 |
| **1-10** comparative | 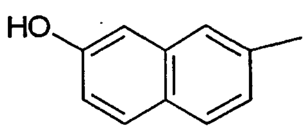 | 0 | 0 | bond | C | 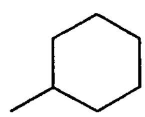 |
| **1-11** | 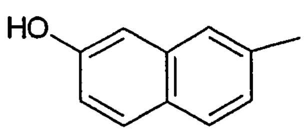 | 0 | 0 | -O- | N | 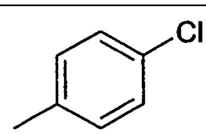 |

(continued)

| Example | A | m | p | -X- | Y | -R1 |
|---|---|---|---|---|---|---|
| **1-12** comparative | 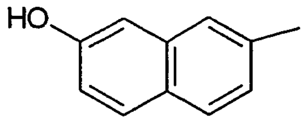 | 0 | 2 | -O- | N | 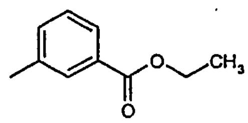 |
| **1-13** | 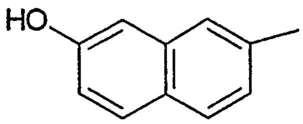 | 0 | 1 | -NH- | N | 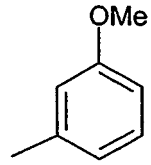 |
| **1-14** | 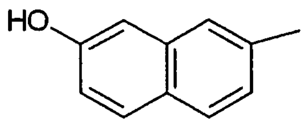 | 1 | 1 | bond | N | 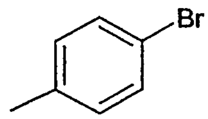 |
| **1-15** comparative | 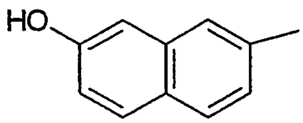 | 1 | 2 | bond | N | 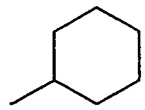 |
| **1-16** comparative | 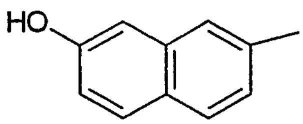 | 1 | 2 | bond | N | 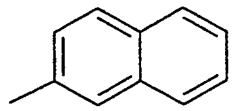 |
| **1-17** comparative | 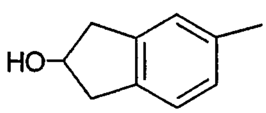 | 1 | 2 | bond | C | 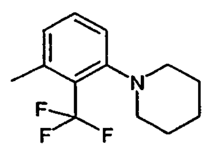 |
| **1-18** comparative | 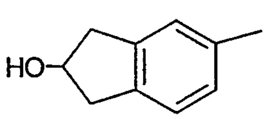 | 0 | 1 | bond | C | 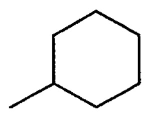 |
| **1-19** | 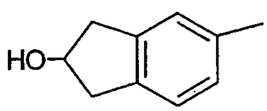 | 0 | 1 | -O- | C | 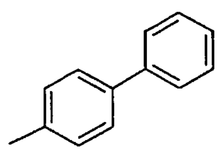 |
| **1-20** comparative | 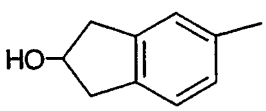 | 0 | 2 | -O- | C | 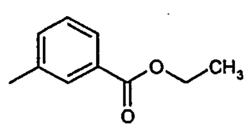 |
| **1-21** | 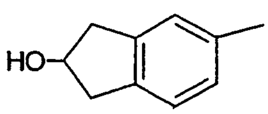 | 0 | 0 | -NH- | C | 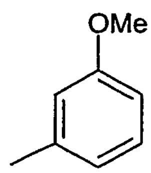 |

(continued)

| Example | A | m | p | -X- | Y | -R1 |
|---|---|---|---|---|---|---|
| **1-22** | 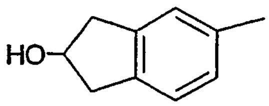 | 1 | 1 | bond | N | 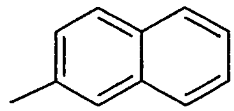 |
| **1-23** comparative | 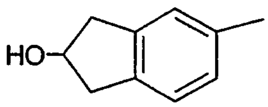 | 1 | 1 | bond | N | 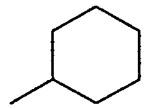 |

## Claims

**1.** A bicyclic amide, carbamate or urea derivative of the formula (I), its tautomeric or stereoisomeric form, or a salt thereof:

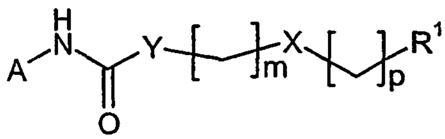

(I)

wherein

A represents

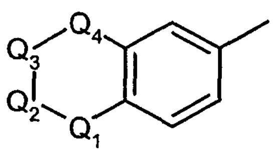 or 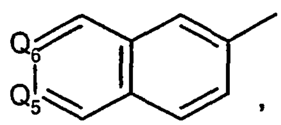 ,

wherein

$Q_1$ and $Q_4$ independently represent direct bond or methylene;
$Q_2$ represents $CHR^2$, or CO,
$Q_3$ represents $CHR^3$, or CO,
wherein

$R^2$ represents hydrogen, hydroxy, $C_{1-6}$ alkoxy, $C_{1-6}$ alkanoyloxy, or $C_{1-6}$ alkyl optionally substituted by hydroxy, $C_{1-6}$ alkoxy, $C_{1-6}$ alkanoyloxy or mono-, di-, or tri- halogen;
$R^3$ represents hydrogen, hydroxy, $C_{1-6}$ alkoxy, $C_{1-6}$ alkanoyloxy, or $C_{1-6}$ alkyl optionally substituted by hydroxy, $C_{1-6}$ alkoxy, $C_{1-6}$ alkanoyloxy or mono-, di-, or tri- halogen;
with the proviso that

$Q_1$ and $Q_4$ can not be direct bond at the same time;
$R^2$ and $R^3$ can not be hydrogen at the same time;
when $Q_1$ represents direct bond,

$R^3$ represents hydroxy, $C_{1-6}$ alkoxy or $C_{1-6}$ alkanoyloxy;

$Q_5$ represents CH or $CR^5$,
wherein

$R^5$ represents hydroxy, $C_{1-6}$ alkoxy, $C_{1-6}$ alkanoyloxy, or $C_{1-6}$ alkyl optionally substituted by hydroxy, $C_{1-6}$ alkoxy, $C_{1-6}$ alkanoyloxy or mono-, di-, or tri- halogen;

$Q_6$ represents CH or $CR^6$,
wherein

$R^6$ represents hydroxy, $C_{1-6}$ alkoxy, $C_{1-6}$ alkanoyloxy, or $C_{1-6}$ alkyl optionally substituted by hydroxy, $C_{1-6}$ alkoxy, $C_{1-6}$ alkanoyloxy or mono-, di-, or tri- halogen;
with the proviso that $Q_5$ and $Q_6$ can not be CH at the same time;

m represents an integer from 0 to 3;
p represents an integer 0 or 1;
-X- represents a bond, -O- or $N(R^4)$-,
-Y- represents $CH_2$, O or NH; and
wherein

$R^4$ represents hydrogen or $C_{1-6}$ alkyl,
with the proviso that when m is 0, -X- represents a bond; and

$R^1$ represents aryl or heteroaryl,
wherein
said aryl and heteroaryl are optionally substituted with one or more substituents independently selected from the group consisting of halogen, nitro, hydroxy, carboxy, cyano, amino, N-($C_{1-6}$alkyl)amino, N,N-di($C_{1-6}$alkyl) amino, N-($C_{3-8}$ cycloalkyl)amino, $C_{1-6}$alkoxycarbonyl, sulfonamide, $C_{1-6}$ alkanoyl, N-($C_{1-6}$alkanoyl)amino, carbamoyl, $C_{1-6}$ alkyl-carbamoyl, $C_{3-8}$cycloalkyl, heterocycle,
$C_{1-6}$ alkyl [wherein said alkyl is optionally substituted by cyano, nitro, hydroxy, carboxy, amino, $C_{1-6}$ alkoxycarbonyl or mono-, di-, or tri-halogen],
$C_{1-6}$ alkoxy [wherein said alkoxy is optionally substituted by mono-, di-, or tri- halogen],
$C_{1-6}$ alkylthio [wherein said alkylthio is optionally substituted by mono-, di-, or tri- halogen],
phenyl, benzyl and phenoxy,
[wherein said phenyl, phenyl moiety of said benzyl or phenyl moiety of said phenoxy are optionally substituted by halogen, nitro, hydroxy, carboxy, amino, N-($C_{1-6}$alkyl)amino, N,N-di($C_{1-6}$ alkyl)amino, N-($C_{3-8}$cycloalkyl) amino, $C_{1-6}$ alkoxycarbonyl, $C_{1-6}$alkoxycarbonyl or $C_{1-6}$ alkyl].

2. The bicyclic amide, carbamate or urea derivative of the formula (I), its tautomeric or stereoisomeric form, or a salt thereof as claimed in claim 1,
wherein

A represents

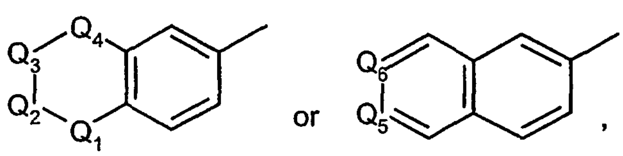

or ,

$Q_1$ and $Q_4$ represent methylene;

$Q_2$ represents $CHR^2$ or CO,
wherein

$R^2$ represents hydrogen, hydroxy, $C_{1-6}$ alkoxy, $C_{1-6}$ alkanoyloxy or $C_{1-6}$ alkyl optionally substituted by mono-, di-, or tri- halogen;

$Q_3$ represents $CHR^3$ or CO,
wherein

$R^3$ represents hydroxy, $C_{1-6}$ alkoxy, $C_{1-6}$ alkanoyloxy, or $C_{1-6}$alkyl optionally substituted by mono-, di-, or tri- halogen;

$Q_5$ represents CH;
$Q_6$ represents $CR^6$,
wherein

$R^6$ represents hydroxy, $C_{1-6}$ alkoxy, $C_{1-6}$ alkanoyloxy, or $C_{1-6}$ alkyl optionally substituted by mono-, di-, or tri- halogen;

m represents an integer from 0 to 3;
p represents an integer 0 or 1;
-X- represents a bond, -O- or $-N(R^4)-$,
wherein

$R^4$ represents hydrogen or $C_{1-6}$ alkyl,
with the proviso that when m is 0, -X- represents a bond;

-Y- represents $CH_2$, O or NH; and
$R^1$ represents phenyl, naphthyl, pyridyl, or pyrimidyl,
wherein
said phenyl, naphthyl, pyridyl or pyrimidyl are optionally substituted with one or more substituents independently selected from the group consisting of halogen, nitro, hydroxy, carboxy, cyano, amino, N-($C_{1-6}$alkyl)amino, N, N-di($C_{1-6}$alkyl)amino, N-($C_{3-8}$ cycloalkyl)amino, $C_{1-6}$alkoxycarbonyl, sulfonamide, $C_{1-6}$ alkanoyl, N-($C_{1-6}$alkanoyl)amino, carbamoyl, $C_{1-6}$ alkylcarbamoyl, $C_{3-8}$cycloalkyl, heterocycle,
$C_{1-6}$ alkyl [wherein said alkyl is optionally substituted by cyano, nitro, hydroxy, carboxy, amino, $C_{1-6}$ alkoxycarbonyl or mono-, di-, or tri-halogen],
$C_{1-6}$ alkoxy [wherein said alkoxy is optionally substituted by mono-, di-, or tri- halogen],
$C_{1-6}$ alkylthio [wherein said alkylthio is optionally substituted by mono-, di-, or tri- halogen],
phenyl, benzyl and phenoxy,
[wherein said phenyl, phenyl moiety of said benzyl or phenyl moiety of said phenoxy are optionally substituted by halogen, nitro, hydroxy, carboxy, amino, N-($C_{1-6}$alkyl)amino, N,N-di($C_{1-6}$ alkyl)amino, N-($C_{3-8}$ cycloalkyl) amino, $C_{1-6}$ alkoxycarbonyl, $C_{1-6}$ alkoxycarbonyl or $C_{1-6}$ alkyl].

**3.** The bicyclic amide, carbamate or urea derivative of the formula (I), its tautomeric or stereoisomeric form, or a salt thereof as claimed in claim 1,
wherein

A represents

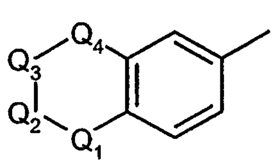

,

$Q_1$ represents methylene;
$Q_4$ represents direct bond;
$Q_2$ represents $CHR^2$ or CO,
wherein

$R^2$ represents hydroxy, $C_{1-6}$ alkoxy or $C_{1-6}$ alkanoyloxy;

$Q_3$ represents $CHR^3$,
wherein

$R^3$ represents hydrogen;

m represents an integer from 0 to 3;
p represents an integer 0 or 1;
-X- represents a bond, -O- or $-N(R^4)-$,
wherein

$R^4$ represents hydrogen or $C_{1-6}$ alkyl,
with the proviso that when m is 0, -X- represents a bond;

-Y- represents $CH_2$, O or NH; and
$R^1$ represents phenyl, naphthyl, pyridyl, or pyrimidyl,
wherein
said phenyl, naphthyl, pyridyl or pyrimidyl are optionally substituted with one or more substituents independently selected from the group consisting of halogen, nitro, hydroxy, carboxy, cyano, amino, N-($C_{1-6}$alkyl)amino, N, N-di($C_{1-6}$alkyl)amino, N-($C_{3-8}$ cycloalkyl)amino, $C_{1-6}$alkoxycarbonyl, sulfonamide, $C_{1-6}$ alkanoyl, N-($C_{1-6}$alkanoyl)amino, carbamoyl, $C_{1-6}$ alkylcarbamoyl, $C_{3-8}$cycloalkyl, heterocycle,
$C_{1-6}$ alkyl [wherein said alkyl is optionally substituted by cyano, nitro, hydroxy, carboxy, amino, $C_{1-6}$ alkoxycarbonyl or mono-, di-, or tri-halogen],
$C_{1-6}$ alkoxy [wherein said alkoxy is optionally substituted by mono-, di-, or tri- halogen],
$C_{1-6}$ alkylthio [wherein said alkylthio is optionally substituted by mono-, di-, or tri- halogen],
phenyl, benzyl and phenoxy ,
[wherein said phenyl, phenyl moiety of said benzyl or phenyl moiety of said phenoxy are optionally substituted by halogen, nitro, hydroxy, carboxy, amino, N-($C_{1-6}$alkyl)amino, N,N-di($C_{1-6}$ alkyl)amino, N-($C_{3-8}$ cycloalkyl) amino, $C_{1-6}$ alkoxycarbonyl, $C_{1-6}$ alkoxycarbonyl or $C_{1-6}$ alkyl].

4. The bicyclic amide, carbamate or urea derivative of the formula (I), its tautomeric or stereoisomeric form, or a salt thereof as claimed in claim 1,
wherein

A represents

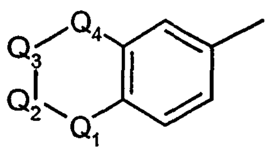

,

$Q_1$ and $Q_4$ represents methylene;
$Q_2$ represents $CHR^2$,
wherein

$R^2$ represents hydrogen;

$Q_3$ represents $CHR^3$,
wherein

m $R^3$ represents hydrogen, hydroxy, $C_{1-6}$ alkoxy or $C_{1-6}$ alkanoyloxy; represents an integer from 0 to 3;
p represents an integer 0 or 1;
-X- represents a bond, -O- or $-N(R^4)-$,
wherein $R^4$ is hydrogen or $C_{1-6}$ alkyl,
with the proviso that when m is 0, -X- represents a bond;
-Y- represents $CH_2$, O or NH; and

$R^1$ represents phenyl, naphthyl, pyridyl, or pyrimidyl,
wherein
said phenyl, naphthyl, pyridyl or pyrimidyl are optionally substituted with one or more substituents independently selected from the group consisting of halogen, nitro, hydroxy, carboxy, cyano, amino, N-($C_{1-6}$alkyl)amino, N, N-di($C_{1-6}$alkyl)amino, N-($C_{3-8}$ cycloalkyl)amino, $C_{1-6}$alkoxycarbonyl, sulfonamide, $C_{1-6}$ alkanoyl, N-($C_{1-6}$alkanoyl)amino, carbamoyl, $C_{1-6}$ alkylcarbamoyl, $C_{3-8}$cycloalkyl, heterocycle,
$C_{1-6}$ alkyl [wherein said alkyl is optionally substituted by cyano, nitro, hydroxy, carboxy, amino, $C_{1-6}$ alkoxycarbonyl or mono-, di-, or tri-halogen],
$C_{1-6}$ alkoxy [wherein said alkoxy is optionally substituted by mono-, di-, or tri- halogen],
$C_{1-6}$ alkylthio [wherein said alkylthio is optionally substituted by mono-, di-, or tri- halogen],
phenyl, benzyl and phenoxy,
[wherein said phenyl, phenyl moiety of said benzyl or phenyl moiety of said phenoxy are optionally substituted by halogen, nitro, hydroxy, carboxy, amino, N-($C_{1-6}$alkyl)amino, N,N-di($C_{1-6}$ alkyl)amino, N-($C_{3-8}$ cycloalkyl) amino, $C_{1-6}$ alkoxycarbonyl, $C_{1-6}$ alkoxycarbonyl or $C_{1-6}$ alkyl].

**5.** The bicyclic amide, carbamate or urea derivative of the formula (I), its tautomeric or stereoisomeric form, or a salt thereof as claimed in claim 1,
wherein

A represents

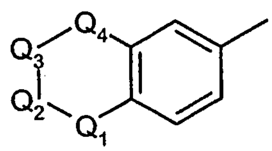
,

$Q_1$ and $Q_4$ represent methylene;
$Q_2$ represents $CHR^2$,
wherein

$R^2$ represents hydroxy, $C_{1-6}$ alkoxy or $C_{1-6}$ alkanoyloxy;

$Q_3$ represents $CHR^3$,
wherein

$R^3$ represents hydrogen;

m represents an integer from 1 to 3;
p represents 0 or 1;
-X- represents a bond, -O- or -N($R^4$)-,
wherein

$R^4$ is hydrogen or $C_{1-6}$ alkyl ,
with the proviso that when m is 0, -X- represents a bond;

-Y- represents $CH_2$, O or NH; and
$R^1$ represents phenyl, naphthyl, pyridyl, or pyrimidyl,
wherein
said phenyl, naphthyl, pyridyl or pyrimidyl are optionally substituted with one or more substituents independently selected from the group consisting of halogen, nitro, hydroxy, carboxy, cyano, amino, N-($C_{1-6}$alkyl)amino, N, N-di($C_{1-6}$alkyl)a,ino, N-($C_{3-8}$ cycloalkyl)amino, $C_{1-6}$alkoxycarbonyl, sulfonamide, $C_{1-6}$ alkanoyl, N-($C_{1-6}$alkanoyl) amino, carbamoyl, $C_{1-6}$ alkylcarbamoyl, $C_{3-8}$cycloalkyl, heterocycle,
$C_{1-6}$ alkyl [wherein said alkyl is optionally substituted by cyano, nitro, hydroxy, carboxy, amino, $C_{1-6}$ alkoxycarbonyl or mono-, di-, or tri-halogen],

$C_{1-6}$ alkoxy [wherein said alkoxy is optionally substituted by mono-, di-, or tri- halogen],
$C_{1-6}$ alkylthio [wherein said alkylthio is optionally substituted by mono-, di-, or tri- halogen],
phenyl, benzyl and phenoxy ,
[wherein said phenyl, phenyl moiety of said benzyl or phenyl moiety of said phenoxy are optionally substituted by halogen, nitro, hydroxy, carboxy, amino, N-($C_{1-6}$alkyl)amino, N,N-di($C_{1-6}$alkl)amino, N-($C_{3-8}$cycloalkyl)amino, $C_{1-6}$alkoxy-carbonyl, $C_{1-6}$ alkoxycarbonyl or $C_{1-6}$ alkyl].

**6.** The bicyclic amide, carbamate or urea derivative of the formula (I), its tautomeric or stereoisomeric form, or a salt thereof as claimed in claim 1,
wherein

A represents

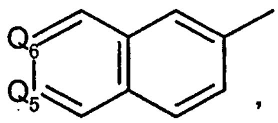
,

$Q_5$ represents CH;
$Q_6$ represent $CR^6$,
wherein

$R^6$ represents hydroxy, $C_{1-6}$ alkoxy, $C_{1-6}$ alkanoyloxy, or $C_{1-6}$ alkyl optionally substituted by hydroxy, $C_{1-6}$ alkoxy or $C_{1-6}$ alkanoyloxy

m represents an integer from 0 to 3;
p represents an integer 0 or 1;
-X- represents a bond, -O- or -N($R^4$)-,
wherein

$R^4$ represents hydrogen or $C_{1-6}$ alkyl,
with the proviso that when m is 0, -X- represents a bond;

-Y- represents NH, O or $CH_2$; and
$R^1$ represents phenyl, naphthyl, pyridyl, or pyrimidyl,
wherein
said phenyl, naphthyl, pyridyl, or pyrimidyl are optionally substituted by one or two of substituents selected from the group consisting of halogen, nitro, $C_{1-6}$alkyl, trifluoro$C_{1-6}$alkyl, $C_{1-6}$alkoxy, trifluoro$C_{1-6}$alkoxy and $C_{1-6}$alkanoylamino.

**7.** The bicyclic amide, carbamate or urea derivative of the formula (I), its tautomeric or stereoisomeric form, or a salt thereof as claimed in claim 1,
wherein

A represents

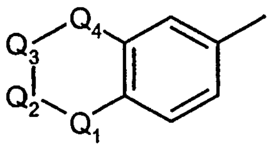
or
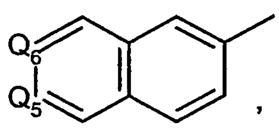
,

$Q_1$ and $Q_4$ represents methylene;
$Q_2$ represents $CHR^2$ ,

wherein

$R^2$ represents hydrogen;

$Q_3$ represents $CHR^3$,
wherein

$R^3$ represents hydrogen, hydroxy, $C_{1-6}$ alkoxy or $C_{1-6}$ alkanoyloxy;

$Q_5$ represents CH;
$Q_6$ represents $CR^6$,
wherein

$R^6$ represents hydroxy;

m represents an integer 2;
p represents an integer 0;
-X- represents a bond, -O- or $-N(R^4)-$,
wherein

$R^4$ is hydrogen or $C_{1-6}$ alkyl,
with the proviso that when m is 0, -X- represents a bond;

-Y- represents NH or O; and
$R^1$ represents phenyl, naphthyl, pyridyl, or pyrimidyl,
wherein
said phenyl, naphthyl, pyridyl, or pyrimidyl are optionally substituted by one or two of substituents selected from the group consisting of chloro, bromo, fluoro, nitro, methyl, methoxy, trifluoromethyl, trifluoroethyl, trifluoromethoxy, trifluoroethoxy, acetamido and propionylamino;

**8.** The bicyclic amide, carbamate or urea derivative of the formula (I), its tautomeric or stereoisomeric form, or a salt thereof as claimed in claim 1, wherein said bicyclic amide, carbamate or urea derivative of the formula (I) is selected from the group consisting of:

N-(7-hydroxy-5,6,7,8-tetrahydronaphthalen-2-yl)-N'-[4-(trifluoromethyl)benzyl]urea;
4-(trifluoromethyl)benzyl(7-hydroxy-5,6,7,8-tetrahydronaphthalen-2-yl)carbamate;
N-(7-hydroxy-5,6,7,8-tetrahydronaphthalen-2-yl)-3-[4-(trifluoromethyl)phenyl]propanamide;
N-(7-hydroxy-5,6,7,8-tetrahydronaphthalen-2-yl)-N'-(2-{[4-(trifluoromethyl)phenyl]-amino}ethyl)urea;
N-(7-hydroxy-5,6,7,8-tetrahydronaphthalen-2-yl)-N'-{2-[4-(trifluoromethyl)phenoxy]-ethyl}urea;
2-{[4-(trifluoromethyl)phenyl]amino}ethyl(7-hydroxy-5,6,7,8-tetrahydronaphthalen-2-yl)carbamate;
2-[4-(trifluoromethyl)phenoxy]ethyl(7-hydroxy-5,6,7,8-tetrahydronaphthalen-2-yl)carbamate;and
N-[4-chloro-3-(trifluoromethyl)phenyl]-N'-(7-hydroxy-5,6,7,8-tetrahydronaphthalen-2-yl)urea;
N-(2-{[4-chloro-3-(trifluoromethyl)phenyl]amino}ethyl)-N'-(7-hydroxy-5,6,7,8-tetrahydro-naphthalen-2-yl)urea;
N-{2-[4-chloro-3-(trifluoromethyl)phenoxy]ethyl}-N'-(7-hydroxy-5,6,7,8-tetrahydro-naphthalen-2-yl)urea;
N-(2-{[4-chloro-3-(trifluoromethyl)phenyl]amino}ethyl)-N'-(6-hydroxy-5,6,7,8-tetrahydronaphthalen-2-yl)urea; and
N-{2-[4-chloro-3-(trifluoromethyl)phenoxy]ethyl}-N'-(6-hydroxy-5,6,7,8-tetrahydronaphthalen-2-yl)urea

**9.** A medicament comprising the bicyclic amide, carbamate or urea derivative of the formula (I), its tautomeric or stereoisomeric form, or a physiologically acceptable salt thereof as claimed in claim 1 as an active ingredient.

**10.** The medicament as claimed in claim 9, further comprising one or more pharmaceutically acceptable excipients.

**11.** The medicament as claimed in claim 9, wherein said bicyclic amide, carbamate or urea derivative of the formula (I), its tautomeric or stereoisomeric form, or a physiologically acceptable salt thereof is a VR1 antagonist.

**12.** The medicament as claimed in claim 9 for the treatment and/or prevention of an urological disorder or disease.

**13.** The medicament as claimed in claim 12, wherein said urological disorder or disease is urge urinary incontinence or overactive bladder.

**14.** The medicament as claimed in claim 9 for the treatment and/or prevention of pain.

**15.** The medicament as claimed in claim 14, wherein said pain is chronic pain, neuropathic pain, postoperative pain, or rheumatoid arthritic pain.

**16.** The medicament as claimed in claim 9 for the treatment and/or prevention of a disorder or disease related to pain.

**17.** The medicament as claimed in claim 16, wherein said disorder or disease related to pain is neuralgia, neuropathies, algesia, nerve injury, ischaemia, neurodegeneration, or stroke.

**18.** The medicament as claimed in claim 9 for the treatment and/or prevention of an inflammatory disorder or disease.

**19.** The medicament as claimed in claim 18, wherein said inflammatory disorder or disease is asthma or COPD.

**20.** Use of compounds according to claim 1 for manufacturing a medicament for the treatment and/or prevention of an urological disorder or disease.

**21.** Use of compounds according to claim 1 for manufacturing a medicament for the treatment and/or prevention of pain.

**22.** Use of compounds according to claim 1 for manufacturing a medicament for the treatment and/or prevention of an inflammatory disorder or disease.

**Patentansprüche**

**1.** Bizyklisches Amid-, Carbamat- oder Harnstoffderivat der Formel (I), dessen tautomere oder stereoisomere Form oder ein Salz davon:

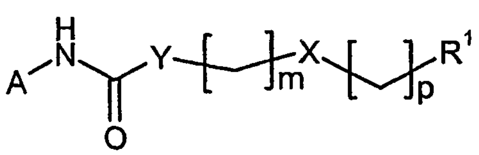

(I)

worin

A für

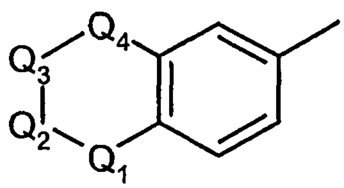

oder

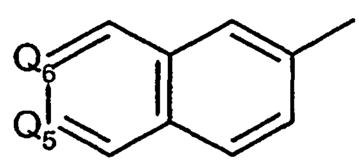

steht,
worin

$Q_1$ und $Q_4$ unabhängig voneinander für eine direkte Bindung oder Methylen stehen;

$Q_2$ für $CHR^2$ oder CO steht,
$Q_3$ für $CHR^3$ oder CO steht,
worin

$R^2$ für Wasserstoff, Hydroxy, $C_{1-6}$-Alkoxy, $C_{1-6}$-Alkanoyloxy oder $C_{1-6}$-Alkyl steht, das gegebenenfalls mit Hydroxy, $C_{1-6}$-Alkoxy, $C_{1-6}$-Alkanoyloxy oder Mono-, Di- oder Trihalogen substituiert ist;
$R^3$ für Wasserstoff, Hydroxy, $C_{1-6}$-Alkoxy, $C_{1-6}$-Alkanoyloxy oder $C_{1-6}$-Alkyl steht, das gegebenenfalls mit Hydroxy, $C_{1-6}$-Alkoxy, $C_{1-6}$-Alkanoyloxy oder Mono-, Di- oder Trihalogen substituiert ist;

mit der Maßgabe, dass

$Q_1$ und $Q_4$ nicht gleichzeitig eine direkte Bindung sein können;
$R^2$ und $R^3$ nicht gleichzeitig Wasserstoff sein können;
wenn $Q_1$ eine direkte Bindung darstellt, $R^3$ für Wasserstoff, $C_{1-6}$-Alkoxy oder $C_{1-6}$-Alkanoyloxy steht;

$Q_5$ für CH oder $CR^5$ steht,
worin

$R^5$ für Hydroxy, $C_{1-6}$-Alkoxy, $C_{1-6}$-Alkanoyloxy oder $C_{1-6}$-Alkyl steht, das gegebenenfalls mit Hydroxy, $C_{1-6}$-Alkoxy, $C_{1-6}$-Alkanoyloxy oder Mono-, Di- oder Trihalogen substituiert ist;

$Q_6$ für CH oder $CR^6$ steht,
worin

$R^6$ für Hydroxy, $C_{1-6}$-Alkoxy, $C_{1-6}$-Alkanoyloxy oder $C_{1-6}$-Alkyl steht, das gegebenenfalls mit Hydroxy, $C_{1-6}$-Alkoxy, $C_{1-6}$-Alkanoyloxy oder Mono-, Di- oder Trihalogen substituiert ist;

mit der Maßgabe, dass $Q_5$ und $Q_6$ nicht gleichzeitig CH sein können;

m für eine ganze Zahl von 0 bis 3 steht;
p für eine ganze Zahl steht, die 0 oder 1 ist;
-X- für eine Bindung, -O- oder -N($R^4$)- steht,
worin

$R^4$ für Wasserstoff oder $C_{1-6}$-Alkyl steht,

mit der Maßgabe, dass, wenn m = 0 ist, -X- für eine Bindung steht; und
-Y- für $CH_2$, O oder NH steht; und
$R^1$ für Aryl oder Heteroaryl steht,
worin
das Aryl und das Heteroaryl gegebenenfalls mit einem oder mehreren Substituenten substituiert sind, die unabhängig voneinander aus der aus folgenden be-stehenden Gruppe ausgewählt sind: Halogen, Nitro, Hydroxy, Carboxy, Cyano, Amino, N-($C_{1-6}$-Alkyl)amino, N,N-Di($C_{1-6}$-alkyl)amino, N-($C_{3-8}$-Cycloalkyl)amino, $C_{1-6}$-Alkoxycarbonyl, Sulfonamid, $C_{1-6}$-Alkanoyl, N-($C_{1-6}$-Alkanoyl)amino, Carbamoyl, $C_{1-6}$-Alkylcarbamoyl, $C_{3-8}$-Cycloalkyl, Heterozyklen,
$C_{1-6}$-Alkyl [worin das Alkyl gegebenenfalls mit Cyano, Nitro, Hydroxy, Carboxy, Amino, $C_{1-6}$-Alkoxycarbonyl oder Mono-, Di- oder Trihalogen substituiert ist],
$C_{1-6}$-Alkoxy [worin das Alkoxy gegebenenfalls mit Mono-, Di- oder Trihalogen substituiert ist],
$C_{1-6}$-Alkylthio [worin das Alkylthio gegebenenfalls mit Mono-, Di- oder Trihalogen substituiert ist],
Phenyl, Benzyl und Phenoxy,
[worin das Phenyl, die Phenylgruppierung des Benzyl- oder die Phenylgruppierung des Phenoxyrests gegebenenfalls mit Halogen, Nitro, Hydroxy, Carboxy, Amino, N-($C_{1-6}$-Alkyl)amino, N,N-Di($C_{1-6}$-alkyl)amino, N-($C_{3-8}$-Cycloalkyl)amino, $C_{1-6}$-Alkoxycarbonyl, $C_{1-6}$-Alkoxycarbonyl oder $C_{1-6}$-Alkyl substituiert ist].

**2.** Bizyklisches Amid-, Carbamat- oder Harnstoffderivat der Formel (I), dessen tautomere oder stereoisomere Form oder ein Salz davon nach Anspruch 1:
worin

A für

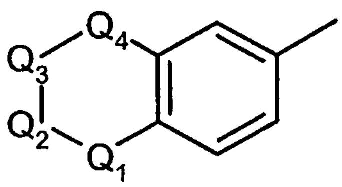

oder

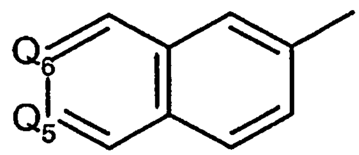

steht,
worin

$Q_1$ und $Q_4$ für Methylen stehen;
$Q_2$ für $CHR^2$ oder CO steht,
worin

$R^2$ für Wasserstoff, Hydroxy, $C_{1-6}$-Alkoxy, $C_{1-6}$-Alkanoyloxy oder $C_{1-6}$-Alkyl steht, das gegebenenfalls mit Mono-, Di- oder Trihalogen substituiert ist;

$Q_3$ für $CHR^3$ oder CO steht,
worin

$R^3$ für Wasserstoff, Hydroxy, $C_{1-6}$-Alkoxy, $C_{1-6}$-Alkanoyloxy oder $C_{1-6}$-Alkyl steht, das gegebenenfalls mit Mono-, Di- oder Trihalogen substituiert ist;

$Q_5$ für CH steht,
$Q_6$ für $CR^6$ steht,
worin

$R^6$ für Hydroxy, $C_{1-6}$-Alkoxy, $C_{1-6}$-Alkanoyloxy oder $C_{1-6}$-Alkyl steht, das gegebenenfalls mit Mono-, Di- oder Trihalogen substituiert ist;

m für eine ganze Zahl von 0 bis 3 steht;
p für eine ganze Zahl steht, die 0 oder 1 ist;
-X- für eine Bindung, -O- oder $-N(R^4)-$ steht,
worin

$R^4$ für Wasserstoff oder $C_{1-6}$-Alkyl steht,
mit der Maßgabe, dass, wenn m = 0 ist, -X- für eine Bindung steht; und

-Y- für $CH_2$, O oder NH steht; und
$R^1$ für Phenyl, Naphthyl, Pyridyl oder Pyrimidyl steht,
worin
das Phenyl, Naphthyl, Pyridyl oder Pyrimidyl gegebenenfalls mit einem oder mehreren Substituenten substituiert ist, die unabhängig voneinander aus der aus folgenden bestehenden Gruppe ausgewählt sind: Halogen, Nitro, Hydroxy, Carboxy, Cyano, Amino, N-($C_{1-6}$-Alkyl)amino, N,N-Di($C_{1-6}$-alkyl)amino, N-($C_{3-8}$-Cycloalkyl)amino, $C_{1-6}$-Alkoxycarbonyl, Sulfonamid, $C_{1-6}$-Alkanoyl, N-($C_{1-6}$-Alkanoyl)amino, Carbamoyl, $C_{1-6}$-Alkylcarbamoyl, $C_{3-8}$-Cycloalkyl, Heterozyklen,
$C_{1-6}$-Alkyl [worin das Alkyl gegebenenfalls mit Cyano, Nitro, Hydroxy, Carboxy, Amino, $C_{1-6}$-Alkoxycarbonyl oder Mono-, Di- oder Trihalogen substituiert ist],
$C_{1-6}$-Alkoxy [worin das Alkoxy gegebenenfalls mit Mono-, Di- oder Trihalogen substituiert ist],
$C_{1-6}$-Alkylthio [worin das Alkylthio gegebenenfalls mit Mono-, Di- oder Trihalogen substituiert ist],
Phenyl, Benzyl und Phenoxy,
[worin das Phenyl, die Phenylgruppierung des Benzyl- oder die Phenylgruppierung des Phenoxyrests gegebenenfalls mit Halogen, Nitro, Hydroxy, Carboxy, Amino, N-($C_{1-6}$-Alkyl)amino, N,N-Di($C_{1-6}$-alkyl)amino, N-($C_{3-8}$-Cycloalkyl)amino, $C_{1-6}$-Alkoxycarbonyl, $C_{1-6}$-Alkoxycarbonyl oder $C_{1-6}$-Alkyl substituiert ist].

**3.** Bizyklisches Amid-, Carbamat- oder Harnstoffderivat der Formel (I), dessen tautomere oder stereoisomere Form oder ein Salz davon nach Anspruch 1:
worin

A für

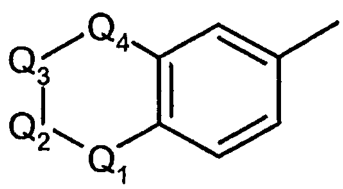

steht,

$Q_1$ für Methylen steht;
$Q_4$ für eine direkte Bindung steht;
$Q_2$ für $CHR^2$ oder CO steht,
worin

$R^2$ für Hydroxy, $C_{1-6}$-Alkoxy oder $C_{1-6}$-Alkanoyloxy steht;

$Q_3$ für $CHR^3$ steht,
worin

$R^3$ für Wasserstoff steht;

m für eine ganze Zahl von 0 bis 3 steht;
p für eine ganze Zahl steht, die 0 oder 1 ist;
-X- für eine Bindung, -O- oder -N($R^4$)- steht,
worin

$R^4$ für Wasserstoff oder $C_{1-6}$-Alkyl steht, mit der Maßgabe, dass, wenn m = 0 ist, -X- für eine Bindung steht; und

-Y- für $CH_2$ oder NH steht; und
$R^1$ für Phenyl, Naphthyl, Pyridyl oder Pyrimidyl steht,
worin
das Phenyl, Naphthyl, Pyridyl oder Pyrimidyl gegebenenfalls mit einem oder mehreren Substituenten substituiert ist, die unabhängig voneinander aus der aus folgenden bestehenden Gruppe ausgewählt sind: Halogen, Nitro, Hydroxy, Carboxy, Cyano, Amino, N-($C_{1-6}$-Alkyl)amino, N,N-Di($C_{1-6}$-alkyl)amino, N-($C_{3-8}$-Cycloalkyl)amino, $C_{1-6}$-Alkoxycarbonyl, Sulfonamid, $C_{1-6}$-Alkanoyl, N-($C_{1-6}$-Alkanoyl)amino, Carbamoyl, $C_{1-6}$-Alkylcarbamoyl, $C_{3-9}$-Cycloalkyl, Heterozyklen,
$C_{1-6}$-Alkyl [worin das Alkyl gegebenenfalls mit Cyano, Nitro, Hydroxy, Carboxy, Amino, $C_{1-6}$-Alkoxycarbonyl oder Mono-, Di- oder Trihalogen substituiert ist],
$C_{1-6}$-Alkoxy [worin das Alkoxy gegebenenfalls mit Mono-, Di- oder Trihalogen substituiert ist],
$C_{1-6}$-Alkylthio [worin das Alkylthio gegebenenfalls mit Mono-, Di- oder Trihalogen substituiert ist],
Phenyl, Benzyl und Phenoxy,
[worin das Phenyl, die Phenylgruppierung des Benzyl- oder die Phenylgruppierung des Phenoxyrests gegebenenfalls mit Halogen, Nitro, Hydroxy, Carboxy, Amino, N-($C_{1-6}$-Alkyl)amino, N,N-Di($C_{1-6}$-alkyl)amino, N-($C_{3-8}$-Cycloalkyl)amino, $C_{1-6}$-Alkoxycarbonyl, $C_{1-6}$-Alkoxycarbonyl oder $C_{1-6}$-Alkyl substituiert ist].

**4.** Bizyklisches Amid-, Carbamat- oder Harnstoffderivat der Formel (I), dessen tautomere oder stereoisomere Form oder ein Salz davon nach Anspruch 1:
worin

A für

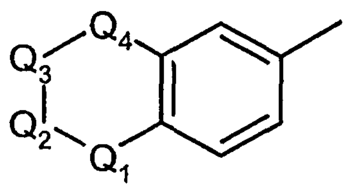

steht,

$Q_1$ und $Q_4$ für Methylen stehen;
$Q_2$ für $CHR^2$ steht,
worin

$R^2$ für Wasserstoff steht;

$Q_3$ für $CHR^3$ steht,
worin

$R^3$ für Wasserstoff, Hydroxy, $C_{1-6}$-Alkoxy oder $C_{1-6}$-Alkanoyloxy steht;

m für eine ganze Zahl von 0 bis 3 steht;
p für eine ganze Zahl steht, die 0 oder 1 ist;
-X- für eine Bindung, -O- oder -N($R^4$)- steht,
worin

$R^4$ für Wasserstoff oder $C_{1-6}$-Alkyl steht,

mit der Maßgabe, dass, wenn m = 0 ist, -X- für eine Bindung steht; und
-Y- für $CH_2$ oder NH steht; und
$R^1$ für Phenyl, Naphthyl, Pyridyl oder Pyrimidyl steht,
worin
das Phenyl, Naphthyl, Pyridyl oder Pyrimidyl gegebenenfalls mit einem oder mehreren Substituenten substituiert ist, die unabhängig voneinander aus der aus folgenden bestehenden Gruppe ausgewählt sind: Halogen, Nitro, Hydroxy, Carboxy, Cyano, Amino, N-($C_{1-6}$-Alkyl)amino, N,N-Di($C_{1-6}$-alkyl)amino, N-($C_{3-8}$-Cycloalkyl)amino, $C_{1-6}$-Alkoxycarbonyl, Sulfonamid, $C_{1-6}$-Alkanoyl, N-($C_{1-6}$-Alkanoyl)amino, Carbamoyl, $C_{1-6}$-Alkylcarbamoyl, $C_{3-8}$-Cycloalkyl, Heterozyklen,
$C_{1-6}$-Alkyl [worin das Alkyl gegebenenfalls mit Cyano, Nitro, Hydroxy, Carboxy, Amino, $C_{1-6}$-Alkoxycarbonyl oder Mono-, Di- oder Trihalogen substituiert ist],
$C_{1-6}$-Alkoxy [worin das Alkoxy gegebenenfalls mit Mono-, Di- oder Trihalogen substituiert ist],
$C_{1-6}$-Alkylthio [worin das Alkylthio gegebenenfalls mit Mono-, Di- oder Trihalogen substituiert ist],
Phenyl, Benzyl und Phenoxy,
[worin das Phenyl, die Phenylgruppierung des Benzyl- oder die Phenylgruppierung des Phenoxyrests gegebenenfalls mit Halogen, Nitro, Hydroxy, Carboxy, Amino, N-($C_{1-6}$-Alkyl)amino, N,N-Di($C_{1-6}$-alkyl)amino, N-($C_{3-8}$-Cycloalkyl)amino, $C_{1-6}$-Alkoxycarbonyl, $C_{1-6}$-Alkoxycarbonyl oder $C_{1-6}$-Alkyl substituiert ist].

**5.** Bizyklisches Amid-, Carbamat- oder Harnstoffderivat der Formel (I), dessen tautomere oder stereoisomere Form oder ein Salz davon nach Anspruch 1:
worin

A für

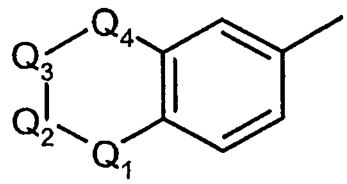

steht,

$Q_1$ und $Q_4$ für Methylen stehen;
$Q_2$ für $CHR^2$ steht,
worin

$R^2$ für Hydroxy, $C_{1-6}$-Alkoxy oder $C_{1-6}$-Alkanoyloxy steht;

$Q_3$ für $CHR^3$ steht,
worin

$R^3$ für Wasserstoff steht;

m für eine ganze Zahl von 1 bis 3 steht;
p für 0 oder 1 steht;
-X- für eine Bindung, -O- oder -N($R^4$)- steht,
worin

$R^4$ Wasserstoff oder $C_{1-6}$-Alkyl ist,

mit der Maßgabe, dass, wenn m = 0 ist, -X- für eine Bindung steht; und
-Y- für $CH_2$, O oder NH steht; und
$R^1$ für Phenyl, Naphthyl, Pyridyl oder Pyrimidyl steht,
worin
das Phenyl, Naphthyl, Pyridyl oder Pyrimidyl gegebenenfalls mit einem oder mehreren Substituenten substituiert ist, die unabhängig voneinander aus der aus folgenden bestehenden Gruppe ausgewählt sind: Halogen, Nitro, Hydroxy, Carboxy, Cyano, Amino, N-($C_{1-6}$-Alkyl)amino, N,N-Di($C_{1-6}$-alkyl)amino, N-($C_{3-8}$-Cycloalkyl)amino, $C_{1-6}$-Alkoxycarbonyl, Sulfonamid, $C_{1-6}$-Alkanoyl, N-($C_{1-6}$-Alkanoyl)amino, Carbamoyl, $C_{1-6}$-Alkylcarbamoyl, $C_{3-8}$-Cycloalkyl, Heterozyklen,
$C_{1-6}$-Alkyl [worin das Alkyl gegebenenfalls mit Cyano, Nitro, Hydroxy, Carboxy, Amino, $C_{1-6}$-Alkoxycarbonyl oder Mono-, Di- oder Trihalogen substituiert ist],
$C_{1-6}$-Alkoxy [worin das Alkoxy gegebenenfalls mit Mono-, Di- oder Trihalogen substituiert ist],
$C_{1-6}$-Alkylthio [worin das Alkylthio gegebenenfalls mit Mono-, Di- oder Trihalogen substituiert ist], Phenyl, Benzyl und Phenoxy,
[worin das Phenyl, die Phenylgruppierung des Benzyl- oder die Phenylgruppierung des Phenoxyrests gegebenenfalls mit Halogen, Nitro, Hydroxy, Carboxy, Amino, N-($C_{1-6}$-Alkyl)amino, N,N-Di($C_{1-6}$-alkyl)amino, N-($C_{3-8}$-Cycloalkyl)amino, $C_{1-6}$-Alkoxycarbonyl, $C_{1-6}$-Alkoxycarbonyl oder $C_{1-6}$-Alkyl substituiert ist].

**6.** Bizyklisches Amid-, Carbamat- oder Harnstoffderivat der Formel (I), dessen tautomere oder stereoisomere Form oder ein Salz davon nach Anspruch 1:
worin

A für

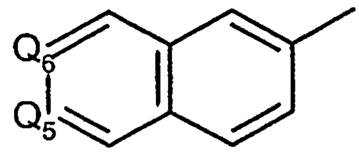

steht,

$Q_5$ für CH steht;
$Q_6$ für $CR^6$ steht;
worin

$R^6$ für Hydroxy, $C_{1-6}$-Alkoxy, $C_{1-6}$-Alkanoyloxy oder $C_{1-6}$-Alkyl steht, das gegebenenfalls mit Hydroxy, $C_{1-6}$-Alkoxy, $C_{1-6}$-Alkanoyloxy substituiert ist;

m für eine ganze Zahl von 0 bis 3 steht;
p für eine ganze Zahl steht, die 0 oder 1 ist;
-X- für eine Bindung, -O- oder -N($R^4$)- steht,
worin

$R^4$ für Wasserstoff oder $C_{1-6}$-Alkyl steht,

mit der Maßgabe, dass, wenn m = 0 ist, -X- für eine Bindung steht; und
-Y- für NH, O oder $CH_2$ steht; und
$R^1$ für Phenyl, Naphthyl, Pyridyl oder Pyrimidyl steht,
worin
das Phenyl, Naphthyl, Pyridyl oder Pyrimidyl gegebenenfalls mit einem oder zwei Substituenten substituiert ist, die unabhängig voneinander aus der aus folgenden bestehenden Gruppe ausgewählt sind: Halogen, Nitro, $C_{1-6}$-Alkyl, Trifluor-$C_{1-6}$-alkyl, $C_{1-6}$-Alkoxy, Trifluor-$C_{1-6}$-alkoxy und $C_{1-6}$-Alkanoylamino.

**7.** Bizyklisches Amid-, Carbamat- oder Harnstoffderivat der Formel (I), dessen tautomere oder stereoisomere Form oder ein Salz davon nach Anspruch 1:
worin

A für

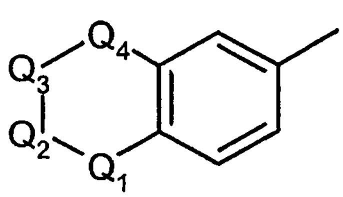

oder

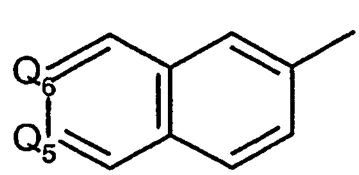

steht,

$Q_1$ und $Q_4$ für Methylen stehen;
$Q_2$ für $CHR^2$ steht,
worin

$R^2$ für Wasserstoff steht;

$Q_3$ für $CHR^3$ steht,
worin

$R^3$ für Wasserstoff, Hydroxy, $C_{1-6}$-Alkoxy oder $C_{1-6}$-Alkanoyloxy steht;

$Q_5$ für CH steht,
$Q_6$ für $CR^6$ steht,
worin

$R^6$ für Hydroxy steht;

m für die ganze Zahl 2 steht;
p für die ganze Zahl 0 steht;
-X- für eine Bindung, -O- oder -N($R^4$)- steht,
worin

$R^4$ für Wasserstoff oder $C_{1-6}$-Alkyl steht,

mit der Maßgabe, dass, wenn m = 0 ist, -X- für eine Bindung steht; und
-Y- für NH oder O steht; und
$R^1$ für Phenyl, Naphthyl, Pyridyl oder Pyrimidyl steht,
worin
das Phenyl, Naphthyl, Pyridyl oder Pyrimidyl gegebenenfalls mit einem oder mehreren Substituenten substituiert ist, die unabhängig voneinander aus der aus folgenden bestehenden Gruppe ausgewählt sind: Chlor, Brom, Fluor, Nitro, Methyl, Methoxy, Trifluormethyl, Trifluorethyl, Trifluormethoxy, Trifluorethoxy, Acetamido und Propionylamino.

**8.** Bizyklisches Amid-, Carbamat- oder Harnstoffderivat der Formel (I), dessen tautomere oder stereoisomere Form oder ein Salz davon nach Anspruch 1, worin das bizyklische Amid-, Carbamat- oder Harnstoffderivat der Formel (I) aus der aus Folgendem bestehenden Gruppe ausgewählt ist:

N-(7-Hydroxy-5,6,7,8-tetrahydronaphthalin-2-yl)-N'-[4-(trifluormethyl)benzyl]harnstoff;
4-(Trifluormethyl)benzyl-(7-hydroxy-5,6,7,8-tetrahydronaphthalin-2-yl)carbamat;
N-(7-Hydroxy-5,6,7,8-tetrahydronaphthalin-2-yl)-3-[4-(trifluormethyl)phenyl]propanamid;
N-(7-Hydroxy-5,6,7,8-tetrahydronaphthalin-2-yl)-N'-(2-{[4-(trifluormethyl)phenyl]-amino}ethyl)harnstoff;
N-(7-Hydroxy-5,6,7,8-tetrahydronaphthalin-2-yl)-N'-{2-[4-(trifluormethyl)phenoxy]-ethyl}harnstoff;
2-{[4-(Trifluormethyl)phenyl]amino}ethyl-(7-hydroxy-5,6,7,8-tetrahydronaphthalin-2-yl)carbamat;
2-[4-(Trifluormethyl)phenoxy]ethyl-(7-hydroxy-5,6,7,8-tetrahydronaphthalin-2-yl)-carbamat;
N-[4-Chlor-3-(trifluormethyl)phenyl]-N'-(7-hydroxy-5,6,7,8-tetrahydronaphthalin-2-yl)-harnstoff;
N-(2-{[4-Chlor-3-(trifluormethyl)phenyl]amino}ethyl)-N'-(7-hydroxy-5,6,7,8-tetrahydronaphthalin-2-yl)harnstoff;
N-{2-[4-Chlor-3-(trifluormethyl)phenoxy]ethyl}-N'-(7-hydroxy-5,6,7,8-tetrahydronaphthalin-2-yl)harnstoff;
N-(2-{[4-Chlor-3-(trifluormethyl)phenyl]amino)ethyl)-N'-(6-hydroxy-5,6,7,8-tetrahydronaphthalin-2-yl)harnstoff;
und
N-{2-[4-Chlor-3-(trifluormethyl)phenoxy]ethyl}-N'-(6-hydroxy-5,6,7,8-tetrahydronaphthalin-2-yl)harnstoff.

**9.** Medikament, das bizyklische Amid-, Carbamat- oder Harnstoffderivat der Formel (I), dessen tautomere oder stereoisomere Form oder ein physiologisch annehmbares Salz davon nach Anspruch 1 als Wirkbestandteil umfassend.

**10.** Medikament nach Anspruch 9, weiters einen oder mehrere pharmazeutisch annehmbare Exzipienten umfassend.

**11.** Medikament nach Anspruch 9, worin das bizyklische Amid-, Carbamat- oder Harnstoffderivat der Formel (I), dessen tautomere oder stereoisomere Form oder ein physiologisch annehmbares Salz davon ein VR1-Antagonist ist.

**12.** Medikament nach Anspruch 9 zur Behandlung eines/einer urologischen Leidens oder Erkrankung und/oder zur Vorbeugung dagegen.

**13.** Medikament nach Anspruch 12, worin das/die urologische Leiden oder Erkrankung Dranghaminkontinenz oder eine überaktive Blase ist.

**14.** Medikament nach Anspruch 9 zur Behandlung von und/oder Vorbeugung gegen Schmerz.

**15.** Medikament nach Anspruch 14, worin der Schmerz chronischer Schmerz, neuropathischer Schmerz, postoperativer Schmerz oder Schmerz bei rheumatoider Arthritis ist.

**16.** Medikament nach Anspruch 9 zur Behandlung eines Leidens oder einer Erkrankung im Zusammenhang mit Schmerz und/oder zur Vorbeugung dagegen.

**17.** Medikament nach Anspruch 16, worin das mit Schmerz verbundene Leiden oder die mit Schmerz verbundene Erkrankung Neuralgie, Neuropathie, Algesie, eine Nervenverletzung, Ischämie, Neurodegeneration oder Schlaganfall ist.

**18.** Medikament nach Anspruch 9 zur Behandlung eines/einer Entzündungsleidens oder -erkrankung und/oder zur Vorbeugung dagegen.

**19.** Medikament nach Anspruch 18, worin das/die Entzündungsleiden oder -erkrankung Asthma oder COPD ist.

**20.** Verwendung von Verbindungen nach Anspruch 1 zur Herstellung eines Medikaments zur Behandlung eines/einer urologischen Leidens oder Erkrankung und/oder zur Vorbeugung dagegen.

**21.** Verwendung von Verbindungen nach Anspruch 1 zur Herstellung eines Medikaments zur Behandlung von Schmerz und/oder zur Vorbeugung dagegen.

**22.** Verwendung von Verbindungen nach Anspruch 1 zur Herstellung eines Medikaments zur Behandlung eines/einer Entzündungsleidens oder -erkrankung und/oder zur Vorbeugung dagegen.

## Revendications

**1.** Dérivé d'amide, de carbamate ou d'urée bicyclique de formule (I), sa forme tautomère ou stéréoisomère, ou un de ses sels :

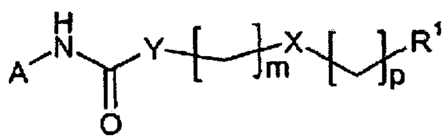

(I)

formule dans laquelle

A représente un groupe

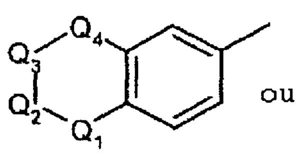

ou

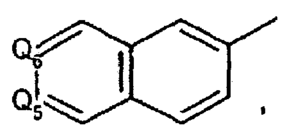

,

dans lequel
$Q_1$ et $Q_4$ représentent indépendamment une liaison directe ou un groupe méthylène ;
$Q_2$ représente un groupe $CHR^2$ ou CO,
$Q_3$ représente un groupe $CHR^3$ ou CO,
où

$R^2$ représente un atome d'hydrogène, un groupe hydroxy, alkoxy en $C_1$ à $C_6$, alcanoyloxy en $C_1$ à $C_6$, ou alkyle en $C_1$ à $C_6$ facultativement substitué avec un substituant hydroxy, alkoxy en $C_1$ à $C_6$, alcanoyloxy en $C_1$ à $C_6$ ou mono-, di- ou trihalogéno ;

$R^3$ représente un atome d'hydrogène, un groupe hydroxy, alkoxy en $C_1$ à $C_6$, alcanoyloxy en $C_1$ à $C_6$, ou alkyle en $C_1$ à $C_6$ facultativement substitué avec un substituant hydroxy, alkoxy en $C_1$ à $C_6$, alcanoyloxy en $C_1$ à $C_6$ ou mono-, di- ou trihalogéno ;

sous réserve que

$Q_1$ et $Q_4$ ne puissent représenter une liaison directe en même temps ;
$R^2$ et $R^3$ ne puissent représenter un atome d'hydrogène en même temps
lorsque $Q_1$ représente une liaison directe,
$R^3$ représente un groupe hydroxy, alkoxy en $C_1$ à $C_6$ ou alcanoyloxy en $C_1$ à $C_6$ ;

$Q_5$ représente un groupe CH ou $CR^5$,
dans lequel

$R^5$ représente un groupe hydroxy, alkoxy en $C_1$ à $C_6$, alcanoyloxy en $C_1$ à $C_6$,
ou alkyle en $C_1$ à $C_6$ facultativement substitué avec un substituant hydroxy, alkoxy en $C_1$ à $C_6$, alcanoyloxy en $C_1$ à $C_6$ ou mono-, di- ou trihalogéno ;

$Q_6$ représente un groupe CH ou $CR^6$,
dans lequel

$R^6$ représente un groupe hydroxy, alkoxy en $C_1$ à $C_6$, alcanoyloxy en $C_1$ à $C_6$,
ou alkyle en $C_1$ à $C_6$ facultativement substitué avec un substituant hydroxy, alkoxy en $C_1$ à $C_6$, alcanoyloxy en $C_1$ à $C_6$ ou mono-, di- ou trihalogéno ;

sous réserve que $Q_5$ et $Q_6$ ne puissent représenter un groupe CH en même temps ;
m représente un nombre entier de 0 à 3 ;
p représente le nombre entier 0 ou 1 ;
-X- représente une liaison, un groupe -O- ou $-N(R^4)-$,
dans lequel

$R^4$ représente un atome d'hydrogène ou un groupe alkyle en $C_1$ à $C_6$,
sous réserve que, lorsque m est égal à 0, -X- représente une liaison ; et

-Y- représente un groupe $CH_2$, O ou un groupe NH ; et
$R^1$ représente un groupe aryle ou hétéroaryle ;
où
lesdits groupes aryle et hétéroaryle sont facultativement substitués avec un ou plusieurs substituants choisis indépendamment dans le groupe consistant en des substituants halogéno, nitro, hydroxy, carboxy, cyano, amino, N-(alkyle en $C_1$ à $C_6$) amino, N,N-di(alkyle en $C_1$ à $C_6$)amino, N-(cycloalkyle en $C_3$ à $C_8$)amino, (alkoxy en $C_1$ à $C_6$) carbonyle, sulfonamide, alcanoyle en $C_1$ à $C_6$, N-(alcanoyle en $C_1$ à $C_6$) amino, carbamoyle, (alkyle en $C_1$ à $C_6$) carbamoyle, cycloalkyle en $C_3$ à $C_8$, hétérocycle, alkyle en $C_1$ à $C_6$ [ledit groupe alkyle étant facultativement substitué avec un substituant cyano, nitro, hydroxy, carboxy, amino, (alkoxy en $C_1$ à $C_6$)carbonyle ou mono-, di- ou trihalogéno],
alkoxy en $C_1$ à $C_6$ [ledit groupe alkoxy étant facultativement substitué avec un substituant mono-, di- ou trihalogéno],
alkylthio en $C_1$ à $C_6$ [ledit groupe alkylthio étant facultativement substitué avec un substituant mono-, di- ou trihalogéno],
phényle, benzyle et phénoxy, [ledit groupe phényle, le groupement phényle dudit groupe benzyle ou le groupement phényle dudit groupe phénoxy est facultativement substitué avec un substituant halogéno, nitro, hydroxy, carboxy, amino, N-(alkyle en $C_1$ à $C_6$)amino, N,N-di(alkyle en $C_1$ à $C_6$) amino, N-(cycloalkyle en $C_3$ à $C_8$)amino, (alkoxy en $C_1$ à $C_6$)carbonyle, (alkoxy en $C_1$ à $C_6$)-carbonyle ou alkyle en $C_1$ à $C_6$].

**2.** Dérivé d'amide, de carbamate ou d'urée bicyclique de formule (I), sa forme tautomère ou stéréoisomère, ou un de ses sels, suivant la revendication 1,
dans lequel

A représente un groupe

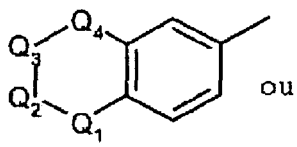 ou 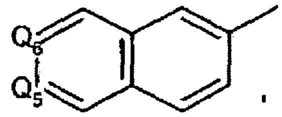 .

$Q_1$ et $Q_4$ représentent des groupes méthylène ;
$Q_2$ représente un groupe $CHR^2$ ou CO,
dans lequel

$R^2$ représente un atome d'hydrogène, un groupe hydroxy, alkoxy en $C_1$ à $C_6$, alcanoyloxy en $C_1$ à $C_6$, ou alkyle en $C_1$ à $C_6$ facultativement substitué avec un substituant mono-, di- ou trihalogéno ;

$Q_3$ représente un groupe $CHR^3$ ou CO,
dans lequel

$R^3$ représente un groupe hydroxy, alkoxy en $C_1$ à $C_6$, alcanoyloxy en $C_1$ à $C_6$, ou alkyle en $C_1$ à $C_6$ facultativement substitué avec un substituant mono-, di- ou trihalogéno ;

$Q_5$ représente un groupe CH,
$Q_6$ représente un groupe $CR^6$,
dans lequel

$R^6$ représente un groupe hydroxy, alkoxy en $C_1$ à $C_6$, alcanoyloxy en $C_1$ à $C_6$, ou alkyle en $C_1$ à $C_6$ facultativement substitué avec un substituant mono-, di- ou trihalogéno ;

m représente un nombre entier de 0 à 3 ;
p représente le nombre entier 0 ou 1 ;
-X- représente une liaison, un groupe -O- ou $-N(R^4)-$,
dans lequel

$R^4$ représente un atome d'hydrogène ou un groupe alkyle en $C_1$ à $C_6$,
sous réserve que, lorsque m est égal à 0, -X- représente une liaison ;

-Y- représente un groupe $CH_2$, O ou un groupe NH ; et
$R^1$ représente un groupe phényle, naphtyle, pyridyle ou pyrimidyle ;
où
ledit groupe phényle, naphtyle, pyridyle ou pyrimidyle est facultativement substitué avec un ou plusieurs substituants choisis indépendamment dans le groupe consistant en des substituants halogéno, nitro, hydroxy, carboxy, cyano, amino, N-(alkyle en $C_1$ à $C_6$) amino, N, N-di (alkyle en $C_1$ à $C_6$) amino, N-(cycloalkyle en $C_3$ à $C_8$) amino, (alkoxy en $C_1$ à $C_6$) carbonyle, sulfonamide, alcanoyle en $C_1$ à $C_6$, N-(alcanoyle en $C_1$ à $C_6$)amino, carbamoyle, (alkyle en $C_1$ à $C_6$)carbamoyle, cycloalkyle en $C_3$ à $C_8$, hétérocycle,
alkyle en $C_1$ à $C_6$ [ledit groupe alkyle étant facultativement substitué avec un substituant cyano, nitro, hydroxy, carboxy, amino, (alkoxy en $C_1$ à $C_6$)carbonyle ou mono-, di- ou trihalogéno],
alkoxy en $C_1$ à $C_6$ [ledit groupe alkoxy étant facultativement substitué avec un substituant mono-, di- ou trihalogéno],
alkylthio en $C_1$ à $C_6$ [ledit groupe alkylthio étant facultativement substitué avec un substituant mono-, di- ou trihalogéno],
phényle, benzyle et phénoxy, [ledit groupe phényle, le groupement phényle dudit groupe benzyle ou le groupement phényle dudit groupe phénoxy étant facultativement substitué avec un substituant halogéno, nitro, hydroxy, carboxy, amino, N-(alkyle en $C_1$ à $C_6$)amino, N,N-di(alkyle en $C_1$ à $C_6$) amino, N-(cycloalkyle en $C_3$ à $C_8$)amino, (alkoxy en $C_1$ à $C_6$)carbonyle, (alkoxy en $C_1$ à $C_6$)-carbonyle ou alkyle en $C_1$ à $C_6$].

**3.** Dérivé d'amide, de carbamate ou d'urée bicyclique de formule (I), sa forme tautomère ou stéréoisomère, ou un de ses sels, suivant la revendication 1,
dans lequel

A représente un groupe

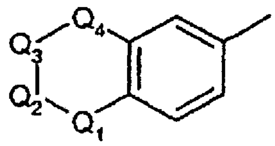

,

$Q_1$ représente un groupe méthylène ;
$Q_4$ représente une liaison directe ;
$Q_2$ représente un groupe $CHR^2$ ou CO,
dans lequel

$R^2$ représente un groupe hydroxy, alkoxy en $C_1$ à $C_6$ ou alcanoyloxy en $C_1$ à $C_6$;

$Q_3$ représente un groupe $CHR^3$,
dans lequel

$R^3$ représente un atome d'hydrogène ;

m représente un nombre entier de 0 à 3 ;
p représente le nombre entier 0 ou 1 ;
-X- représente une liaison, un groupe -O- ou -N($R^4$)-,
dans lequel
$R^4$ représente un atome d'hydrogène ou un groupe alkyle en $C_1$ à $C_6$,
sous réserve que, lorsque m est égal à 0, -X- représente une liaison ;
-Y- représente un groupe $CH_2$, O ou un groupe NH ; et
$R^1$ représente un groupe phényle, naphtyle, pyridyle ou pyrimidyle ;
où
ledit groupe phényle, naphtyle, pyridyle ou pyrimidyle est facultativement substitué avec un ou plusieurs substituants choisis indépendamment dans le groupe consistant en des substituants halogéno, nitro, hydroxy, carboxy, cyano, amino, N-(alkyle en $C_1$ à $C_6$)amino, N,N-di(alkyle en $C_1$ à $C_6$)amino, N-(cycloalkyle en $C_3$ à $C_8$) amino, (alkoxy en $C_1$ à $C_6$)carbonyle, sulfonamide, alcanoyle en $C_1$ à $C_6$, N-(alcanoyle en $C_1$ à $C_6$)amino, carbamoyle, (alkyle en $C_1$ à $C_6$)carbamoyle, cycloalkyle en $C_3$ à $C_8$, hétérocycle,
alkyle en $C_1$ à $C_6$ [ledit groupe alkyle étant facultativement substitué avec un substituant cyano, nitro, hydroxy, carboxy, amino, (alkoxy en $C_1$ à $C_6$)carbonyle ou mono-, di- ou trihalogéno],
alkoxy en $C_1$ à $C_6$ [ledit groupe alkoxy étant facultativement substitué avec un substituant mono-, di- ou trihalogéno],
alkylthio en $C_1$ à $C_6$ [ledit groupe alkylthio étant facultativement substitué avec un substituant mono-, di- ou trihalogéno], phényle, benzyle et phénoxy,
[ledit groupe phényle, le groupement phényle dudit groupe benzyle ou le groupement phényle dudit groupe phénoxy étant facultativement substitué avec un substituant halogéno, nitro, hydroxy, carboxy, amino, N-(alkyle en $C_1$ à $C_6$)amino, N,N-di(alkyle en $C_1$ à $C_6$)amino, N-(cycloalkyle en $C_3$ à $C_8$)amino, (alkoxy en $C_1$ à $C_6$) carbonyle, (alkoxy en $C_1$ à $C_6$)-carbonyle ou alkyle en $C_1$ à $C_6$].

**4.** Dérivé d'amide, de carbamate ou d'urée bicyclique de formule (I), sa forme tautomère ou stéréoisomère, ou un de ses sels, suivant la revendication 1,
dans lequel

A représente un groupe

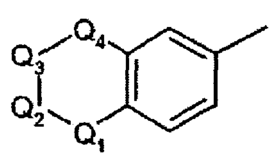

,

$Q_1$ et $Q_4$ représentent des groupes méthylène ;
$Q_2$ représente un groupe $CHR^2$,
dans lequel

$R^2$ représente un atome d'hydrogène ;

$Q_3$ représente un groupe $CHR^3$,
dans lequel

$R^3$ représente un atome d'hydrogène, un groupe hydroxy, alkoxy en $C_1$ à $C_6$ ou alcanoyloxy en $C_1$ à $C_6$;

m représente un nombre entier de 0 à 3 ;
p représente le nombre entier 0 ou 1 ;
-X- représente une liaison, un groupe -O- ou $-N(R^4)-$,
dans lequel $R^4$ représente un atome d'hydrogène ou un groupe alkyle en $C_1$ à $C_6$,
sous réserve que, lorsque m est égal à 0, -X-représente une liaison ;
-Y- représente un groupe $CH_2$, O ou un groupe NH ; et
$R^1$ représente un groupe phényle, naphtyle, pyridyle ou pyrimidyle ;
où ledit groupe phényle, naphtyle, pyridyle ou pyrimidyle est facultativement substitué avec un ou plusieurs substituants choisis indépendamment dans le groupe consistant en des substituants halogéno, nitro, hydroxy, carboxy, cyano, amino, N-(alkyle en $C_1$ à $C_6$)amino, N,N-di(alkyle en $C_1$ à $C_6$)amino, N-(cycloalkyle en $C_3$ à $C_8$)amino, (alkoxy en $C_1$ à $C_6$)carbonyle, sulfonamide, alcanoyle en $C_1$ à $C_6$, N-(alcanoyle en $C_1$ à $C_6$)amino, carbamoyle, (alkyle en $C_1$ à $C_6$)carbamoyle, cycloalkyle en $C_3$ à $C_8$, hétérocycle,
alkyle en $C_1$ à $C_6$ [ledit groupe alkyle étant facultativement substitué avec un substituant cyano, nitro, hydroxy, carboxy, amino, (alkoxy en $C_1$ à $C_6$)carbonyle ou mono-, di- ou trihalogéno],
alkoxy en $C_1$ à $C_6$ [ledit groupe alkoxy étant facultativement substitué avec un substituant mono-, di- ou tri-halogéno],
alkylthio en $C_1$ à $C_6$ [ledit groupe alkylthio étant facultativement substitué avec un substituant mono-, di- ou trihalogéno],
phényle, benzyle et phénoxy, [ledit groupe phényle, le groupement phényle dudit groupe benzyle ou le groupement phényle dudit groupe phénoxy étant facultativement substitué avec un substituant halogéno, nitro, hydroxy, carboxy, amino, N-(alkyle en $C_1$ à $C_6$)amino, N,N-di(alkyle en $C_1$ à $C_6$)amino, N-(cycloalkyle en $C_3$ à $C_8$)amino, (alkoxy en $C_1$ à $C_6$)carbonyle, (alkoxy en $C_1$ à $C_6$)-carbonyle ou alkyle en $C_1$ à $C_6$].

**5.** Dérivé d'amide, de carbamate ou d'urée bicyclique de formule (I), sa forme tautomère ou stéréoisomère, ou un de ses sels, suivant la revendication 1,
dans lequel

A représente un groupe

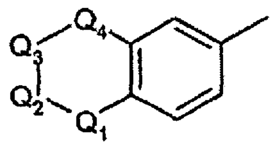
,

$Q_1$ et $Q_4$ représentent des groupes méthylène ;
$Q_2$ représente un groupe $CHR^2$,
dans lequel

$R^2$ représente un groupe hydroxy, alkoxy en $C_1$ à $C_6$ ou alcanoyloxy en $C_1$ à $C_6$ ;

$Q_3$ représente un groupe $CHR^3$,
dans lequel

$R^3$ représente un atome d'hydrogène ;

m représente un nombre entier de 1 à 3 ;
p est égal à 0 ou 1 ;
-X- représente une liaison, un groupe -O- ou -N($R^4$)-,
dans lequel

$R^4$ représente un atome d'hydrogène ou un groupe alkyle en $C_1$ à $C_6$,
sous réserve que, lorsque m est égal à 0, -X- représente une liaison ;

-Y- représente un groupe $CH_2$, O ou un groupe NH ; et
$R^1$ représente un groupe phényle, naphtyle, pyridyle ou pyrimidyle ;
où
ledit groupe phényle, naphtyle, pyridyle ou pyrimidyle est facultativement substitué avec un ou plusieurs substituants choisis indépendamment dans le groupe consistant en des substituants halogéno, nitro, hydroxy, carboxy, cyano, amino, N-(alkyle en $C_1$ à $C_6$)amino, N,N-di(alkyle en $C_1$ à $C_6$)amino, N-(cycloalkyle en $C_3$ à $C_8$) amino, (alkoxy en $C_1$ à $C_6$)carbonyle, sulfonamide, alcanoyle en $C_1$ à $C_6$, N-(alcanoyle en $C_1$ à $C_6$)amino, carbamoyle, (alkyle en $C_1$ à $C_6$)carbamoyle, cycloalkyle en $C_3$ à $C_8$, hétérocycle,
alkyle en $C_1$ à $C_6$ [ledit groupe alkyle étant facultativement substitué avec un substituant cyano, nitro, hydroxy, carboxy, amino, (alkoxy en $C_1$ à $C_6$)carbonyle ou mono-, di- ou trihalogéno],
alkoxy en $C_1$ à $C_6$ [ledit groupe alkoxy étant facultativement substitué avec un substituant mono-, di- ou trihalogéno],
alkylthio en $C_1$ à $C_6$ [ledit groupe alkylthio étant facultativement substitué avec un substituant mono-, di- ou trihalogéno],
phényle, benzyle et phénoxy, [ledit groupe phényle, le groupement phényle dudit groupe benzyle ou le groupement phényle dudit groupe phénoxy étant facultativement substitué avec un substituant halogéno, nitro, hydroxy, carboxy, amino, N-(alkyle en $C_1$ à $C_6$)amino, N,N-di(alkyle en $C_1$ à $C_6$)amino, N-(cycloalkyle en $C_3$ à $C_8$)amino, (alkoxy en $C_1$ à $C_6$)carbonyle, (alkoxy en $C_1$ à $C_6$)-carbonyle ou alkyle en $C_1$ à $C_6$].

**6.** Dérivé d'amide, de carbamate ou d'urée bicyclique de formule (I), sa forme tautomère ou stéréoisomère, ou un de ses sels, suivant la revendication 1,
dans lequel

A représente un groupe

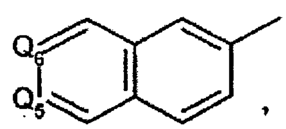

$Q_5$ représente un groupe CH,
$Q_6$ représente un groupe $CR^6$,
dans lequel

$R^6$ représente un groupe hydroxy, alkoxy en $C_1$ à $C_6$, alcanoyloxy en $C_1$ à $C_6$, ou alkyle en $C_1$ à $C_6$ facultativement substitué avec un substituant hydroxy, alkoxy en $C_1$ à $C_6$ ou alcanoyloxy en $C_1$ à $C_6$ ;

m représente un nombre entier de 0 à 3 ;
p représente le nombre entier 0 ou 1 ;
-X- représente une liaison, un groupe -O- ou -N($R^4$)-,
dans lequel

$R^4$ représente un atome d'hydrogène ou un groupe alkyle en $C_1$ à $C_6$,
sous réserve que, lorsque m est égal à 0, -X- représente une liaison ;

-Y- représente un groupe NH, O ou un groupe $CH_2$ ; et
$R^1$ représente un groupe phényle, naphtyle, pyridyle ou pyrimidyle ;
où

ledit groupe phényle, naphtyle, pyridyle ou pyrimidyle est facultativement substitué avec un ou deux substituants choisis dans le groupe consistant en des substituants halogéno, nitro, alkyle en $C_1$ à $C_6$, trifluoralkyle en $C_1$ à $C_6$, alkoxy en $C_1$ à $C_6$, trifluoralkoxy en $C_1$ à $C_6$, et alcanoylamino en $C_1$ à $C_6$.

**7.** Dérivé d'amide, de carbamate ou d'urée bicyclique de formule (I), sa forme tautomère ou stéréoisomère, ou un de ses sels, suivant la revendication 1,
dans lequel

A représente un groupe

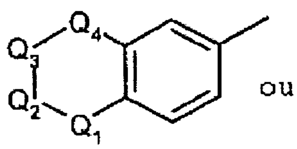 ou 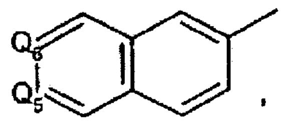 ,

$Q_1$ et $Q_4$ représentent des groupes méthylène ;
$Q_2$ représente un groupe $CHR^2$,
dans lequel

$R^2$ représente un atome d'hydrogène ;

$Q_3$ représente un groupe $CHR^3$,
dans lequel

$R^3$ représente un atome d'hydrogène, un groupe hydroxy, alkoxy en $C_1$ à $C_6$ ou alcanoyloxy en $C_1$ à $C_6$;

$Q_5$ représente un groupe CH,
$Q_6$ représente un groupe $CR^6$,
dans lequel

$R^6$ représente un groupe hydroxy ;

m représente le nombre entier 2 ;
p représente le nombre entier 0 ;
-X- représente une liaison, un groupe -O- ou $-N(R^4)-$,
dans lequel

$R^4$ représente un atome d'hydrogène ou un groupe alkyle en $C_1$ à $C_6$,
sous réserve que, lorsque m est égal à 0, -X- représente une liaison ;

-Y- représente un groupe NH ou O ; et
$R^1$ représente un groupe phényle, naphtyle, pyridyle ou pyrimidyle ;
où
ledit groupe phényle, naphtyle, pyridyle ou pyrimidyle est facultativement substitué avec un ou deux substituants choisis dans le groupe consistant en des substituants chloro, bromo, fluoro, nitro, méthyle, méthoxy, trifluorométhyle, trifluoréthyle, trifluorométhoxy, trifluoréthoxy, acétamido et propionylamino.

**8.** Dérivé d'amide, de carbamate ou d'urée bicyclique de formule (I), sa forme tautomère ou stéréoisomère, ou un de ses sels, suivant la revendication 1, ledit dérivé d'amide, de carbamate ou d'urée bicyclique de formule (I) étant choisi dans le groupe consistant en :

la N-(7-hydroxy-5,6,7,8-tétrahydronaphtalène-2-yl)-N'-[4-(trifluorométhyl)benzyl]urée ;
le 4-(trifluorométhyl)benzyl(7-hydroxy-5,6,7,8-tétra-hydronaphtalène-2-yl)carbamate ;
le N-(7-hydroxy-5,6,7,8-tétrahydronaphtalène-2-yl)-3-[4-(trifluorométhyl)benzyl]propanamide ;
la N-(7-hydroxy-5,6,7,8-tétrahydronaphtalène-2-yl)-N'-(2-{[4-(trifluorométhyl)phényl]amino}éthyl)urée ;
la N-(7-hydroxy-5,6,7,8-tétrahydronaphtalène-2-yl)-N'-{2-[4-(trifluorométhoxy]éthyl}urée ;

le 2-{[4-(trifluorométhyl)phényl]amino}éthyl-(7-hydroxy-5,6,7,8-tétrahydronaphtalène-2-yl)carbamate ;
le 2-[4-(trifluorométhyl)phénoxy]éthyl-(7-hydroxy-5,6,7,8-tétrahydronaphtalène-2-yl)carbamate ; et
la N-[4-chloro-3-(trifluorométhyl)phényl]-N'-(7-hydroxy-5,6,7,8-tétrahydronaphtalène-2-yl)urée ;
la N-(2-{[4-chloro-3-(trifluorométhyl)phényl]amino}éthyl)-N'-(7-hydroxy-5,6,7,8-tétrahydronaphtalène-2-yl) urée ;
la N-{2-[4-chloro-3-(trifluorométhyl)phénoxy]éthyl}-N'-(7-hydroxy-5,6,7,8-tétrahydronaphtalène-2-yl)urée ;
la N-(2-{[4-chloro-3-(trifluorométhyl)phényl]amino}éthyl)-N'-(6-hydroxy-5,6,7,8-tétrahydronaphtalène-2-yl) urée ; et
la N-{2-[4-chloro-3-(trifluorométhyl)phénoxy]éthyl}-N'-(6-hydroxy-5,6,7,8-tétrahydronaphtalène-2-yl)urée.

**9.** Médicament comprenant le dérivé d'amide, de carbamate ou d'urée bicyclique de formule (I), sa forme tautomère ou stéréoisomère, ou un de ses sels physiologiquement acceptables, suivant la revendication 1 comme ingrédient actif.

**10.** Médicament suivant la revendication 9, comprenant en outre un ou plusieurs excipients pharmaceutiquement acceptables.

**11.** Médicament suivant la revendication 9, dans lequel ledit dérivé d'amide, de carbamate ou d'urée bicyclique de formule (I), sa forme tautomère ou stéréoisomère, ou un de ses sels physiologiquement acceptables est un antagoniste de VR1.

**12.** Médicament suivant la revendication 9, destiné au traitement et/ou la prévention d'un trouble ou d'une maladie urologique.

**13.** Médicament suivant la revendication 12, dans lequel ledit trouble ou ladite maladie urologique est l'incontinence urinaire impulsive ou l'hyperactivité vésicale.

**14.** Médicament suivant la revendication 9, destiné au traitement et/ou la prévention de la douleur.

**15.** Médicament suivant la revendication 14, dans lequel ladite douleur est la douleur chronique, la douleur neuropathique, la douleur postopératoire ou la douleur due à la polyarthrite rhumatoïde.

**16.** Médicament suivant la revendication 9, destiné au traitement et/ou la prévention d'un trouble ou d'une maladie en rapport avec la douleur.

**17.** Médicament suivant la revendication 16, dans lequel ledit trouble ou ladite maladie en rapport avec la douleur consiste en les névralgies, les neuropathies, l'algésie, la lésion d'un nerf, l'ischémie, la neurodégénérescence ou l'ictus.

**18.** Médicament suivant la revendication 9, destiné au traitement et/ou la prévention d'un trouble ou d'une maladie inflammatoire.

**19.** Médicament suivant la revendication 18, dans lequel ledit trouble ou ladite maladie inflammatoire est l'asthme ou la maladie pulmonaire obstructive chronique (COPD).

**20.** Utilisation de composés suivant la revendication 1, pour la production d'un médicament destiné au traitement et/ou à la prévention d'un trouble ou d'une maladie urologique.

**21.** Utilisation de composés suivant la revendication 1, pour la production d'un médicament destiné au traitement et/ou à la prévention de la douleur.

**22.** Utilisation de composés suivant la revendication 1, pour la production d'un médicament destiné au traitement et/ou à la prévention d'un trouble ou d'une maladie inflammatoire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9900115 A
- WO 0050387 A
- WO 03014064 A
- WO 03022809 A
- WO 03053945 A
- WO 03070247 A
- WO 03080578 A
- WO 0029577 A

### Non-patent literature cited in the description


- **TOMINAGA M ; CATERINA MJ ; MALMBERG AB ; ROSEN TA ; GILBERT H ; SKINNER K ; RAUMANN BE ; BASBAUM AI ; JULIUS D.** The cloned capsaicin receptor integrates multiple pain-producing stimuli. *Neuron,* 1998, vol. 21, 531-543
- **CATERINA MJ ; SCHUMACHER MA ; TOMINAGA M ; ROSEN TA ; LEVINE JD ; JULIUS D.** *Nature,* 1997, vol. 389, 816-824
- **MAGGI CA.** Therapeutic potential of capsaicin-like molecules - Studies in animals and humans. *Life Sciences,* 1992, vol. 51, 1777-1781
- **DERIDDER D ; CHANDIRAMANI V ; DASGUPTA P ; VANPOPPEL H ; BAERT L ; FOWLER CJ.** Intravesical capsaicin as a treatment for refractory detrusor hyperreflexia: A dual center study with long-term follow-up. *J. Urol.,* 1997, vol. 158, 2087-2092
- **GREENE ; WUTS.** Protective Groups in Organic Synthesis (3rd Edition. John Wiley and Sons, 1999
- **MAGGI CA et al.** *Br.J.Pharmacol.,* 1993, vol. 108, 801-805
- **LECCI A et al.** *Eur. J. Pharmacol.,* 1994, vol. 259, 129-135
- **BENNETT ; XIE.** *Pain,* 1988, vol. 33, 87-107
- **SELTZER et al.** *Pain,* 1990, vol. 43, 205-218
- **KIM SH ; CHUNG JM.** AN EXPERIMENTAL-MODEL FOR PERIPHERAL NEUROPATHY PRODUCED BY SEGMENTAL SPINAL NERVE LIGATION IN THE RA. *PAIN,* 1992, vol. 50 (3), 355-363
- **KLAPDOR et al.** A low cost method to analyse footprint patterns. *J. Neurosci. Methods,* 1997, vol. 75, 49-54